# EUROPEAN PATENT SPECIFICATION

(11) **EP 2 230 519 B1**
(45) Date of publication and mention of the grant of the patent: **14.05.2014**
(21) Application number: 10166054.6
(22) Date of filing: 12.11.2008
(51) Int. Cl.: G01N 33/68

(54) **Biomarkers and methods for diagnosing, predicting and/or prognosing sepsis and uses thereof**
Biomarker und Verfahren zur Diagnose, Vorhersage und/oder Prognose von Sepsis und Verwendungen davon
Biomarqueurs et procédés de diagnostic, de prévision et/ou de pronostic de septicémie et leurs utilisations

(30) Priority: 16.11.2007 EP 07120929
(43) Date of publication of application: 22.09.2010
(62) Divisional of application: 08850110.1
(73) Proprietor: Pronota NV, 9052 Zwijnaarde (BE)
(72) Inventor: Kas, Koen, 2970 Schilde (BE); Vanpoucke, Griet, 9820 Merelbeke (BE); Degroeve, Sven, 2060 Antwerpen (BE); Huijben, Kathleen, 9340 Lede (BE)
(74) Representative: Vanhalst, Koen

(56) References cited:
- WO-A1-2009/061918
- US-A1- 2004 096 917
- ROTH G ET AL: "Susceptibility to programmed cell death in T-lymphocytes from septic patients: a mechanism for lymphopenia and Th2 predominance" BIOCHEMICAL AND BIOPHYSICAL RESEARCH COMMUNICATIONS, ACADEMIC PRESS INC. ORLANDO, FL, US LNKD- DOI:10.1016/S0006-291X(03)01482-7, vol. 308, no. 4, 5 September 2003 (2003-09-05), pages 840-846, XP004447189 ISSN: 0006-291X
- SAFFARZADEH MONA ET AL: "Neutrophil extracellular traps directly induce epithelial and endothelial cell death: a predominant role of histones.", PLOS ONE 2012, vol. 7, no. 2, 2012, page e32366, ISSN: 1932-6203
- FUCHS TOBIAS A ET AL: "Extracellular DNA traps promote thrombosis", PROCEEDINGS OF THE NATIONAL ACADEMY OF SCIENCES OF THE UNITED STATES OF AMERICA, vol. 107, no. 36, September 2010 (2010-09) , pages 15880-15885, ISSN: 0027-8424
- XU JUN ET AL: "Extracellular Histones Are Mediators of Death through TLR2 and TLR4 in Mouse Fatal Liver Injury", JOURNAL OF IMMUNOLOGY, vol. 187, no. 5, September 2011 (2011-09), pages 2626-2631, ISSN: 0022-1767
- XU JUN ET AL: "Extracellular histones are major mediators of death in sepsis.", NATURE MEDICINE NOV 2009, vol. 15, no. 11, November 2009 (2009-11), pages 1318-1321, ISSN: 1546-170X

### SEQUENCE LISTING

<110> PRONOTA N.V.
<120> BIOMARKERS AND METHODS FOR DIAGNOSING, PREDICTING AND/OR PROGNOSING SEPSIS AND USES THEREOF
<130> PRNO-019-EP-DIV
<150> 08850110.1
   <151> 2008-11-12
<150> 07120929.0
   <151> 2007-11-16
<160> 8
<170> PatentIn version 3.3
<210> 1
   <211> 24
   <212> PRT
   <213> Artificial
<220>
   <223> Peptide
<220>
   <221> MOD_RES
   <222> (1)..(1)
   <223> ACETYLATION
<400> 1
   Ser Glu Thr Ala Pro Ala Ala Pro Ala Ala Pro Ala Pro Ala Glu Lys
   1               5                   10                  15
   Thr Pro Val Lys Lys Lys Ala Arg
               20
<210> 2
   <211> 9
   <212> PRT
   <213> Artificial
<220>
   <223> Peptide
<400> 2
   Ala Gly Leu Gln Phe Pro Val Gly Arg
   1               5
<210> 3
   <211> 13
   <212> PRT
   <213> Artificial
<220>
   <223> Peptide
<220>
   <221> MOD_RES
   <222> (1)..(1)
   <223> ACETYLATION
<400> 3
   Ala Val Thr Lys Ala Gln Lys Lys Asp Gly Lys Lys Arg
   1               5                   10
<210> 4
   <211> 13
   <212> PRT
   <213> Artificial
<220>
   <223> Peptide
<220>
   <221> MOD_RES
   <222> (1)..(1)
   <223> ACETYLATION
<400> 4
   Ser Ala Pro Ala Thr Gly Gly Val Lys Lys Pro His Arg
   1               5                   10
<210> 5
   <211> 10
   <212> PRT
   <213> Artificial
<220>
   <223> Peptide
<220>
   <221> MOD_RES
   <222> (1)..(1)
   <223> ACETYLATION
<400> 5
   Tyr Gln Lys Ser Thr Glu Leu Leu Ile Arg
   1 5 10
<210> 6
   <211> 11
   <212> PRT
   <213> Artificial
<220>
   <223> Peptide
<220>
   <221> MOD_RES
   <222> (1)..(1)
   <223> Pyro-glu
<400> 6
   Glu Ile Ala Gln Asp Phe Lys Thr Asp Leu Arg
   1 5 10
<210> 7
   <211> 9
   <212> PRT
   <213> Artificial
<220>
   <223> Peptide
<220>
   <221> MOD_RES
   <222> (1)..(1)
   <223> ACETYLATION
<400> 7
   Lys Ser Thr Gly Gly Lys Ala Pro Arg
   1 5
<210> 8
   <211> 12
   <212> PRT
   <213> Artificial
<220>
   <223> Peptide
<400> 8
   Asp Asn Ile Gln Gly Ile Thr Lys Pro Ala Ile Arg
   1 5 10

## Description

### Field of the invention

The invention relates to protein and/or peptide based biomarkers and molecules binding thereto for diagnosis of disease or determination of a particular condition in a subject, in particular certain peptides or proteins as biomarkers for sepsis and methods for use of the same in diagnosing, prognosing and/or predicting onset of sepsis including methods involving determining increased, decreased or altered expression of said biomarkers in a sample of a subject.

### Background to the invention

In many diseases and conditions, a positive outcome of treatment and/or prophylaxis is strongly correlated with early and/or accurate diagnosis of the disease or condition. However, often there are no effective methods of early diagnosis and treatments are therefore often administered too late, inappropriately or to individuals who will not benefit from it. As a result, many drugs that may be beneficial for some patients may work poorly, not at all, or with adverse effect in other patients. Thus, there is a need for innovative strategies that will allow early detection, prediction, prognosis, diagnosis and treatment of diseases and other biological conditions. There is also a need to determine the ability, or inability, of a patient to tolerate medications or treatments.

Sepsis is more commonly called a blood stream infection or blood poisoning. It is the presence of bacteria (bacteremia), infectious organisms, or their toxins in the blood or other tissues of the body. Sepsis often occurs in patients suffering from systemic inflammatory response syndrome (SIRS), as a result of e.g. surgery, trauma, burns, pancreatitis and other non-infectious events that cause inflammation to occur. SIRS combined with an infection is called sepsis and can occur in many different stages of severity. The infection can occur simultaneously with the occurrence of SIRS e.g. due to infection of a wound or trauma or can occur later due to the latent presence of an infectious organism. Sepsis may be associated with clinical symptoms of systemic (bodywide) illness, such as fever, chills, malaise, low blood pressure, and mental status changes. Sepsis can be a serious situation, a life threatening disease calling for urgent and comprehensive care. Treatment depends on the type of infection, but usually begins with antibiotics or similar medications.

As sepsis may be the result of infection by a wide variety of organisms it is a condition which is particularly difficult to predict and diagnose early enough for effective intervention. It is an excessive and uncontrolled inflammatory response in an individual usually resulting from an individual's inappropriate immune system response to a pathogenic organism. Moreover, there may not be significant numbers of organisms at accessible sites or in body fluids of the affected individual, thus increasing the difficulty of diagnosis. There is therefore a need to identify biomarkers indicating the risk, or early onset of sepsis, regardless of the causative agent, to allow early and effective intervention. Differentiating between patients who are at risk of developing sepsis and those who are not, will also assist in managing the disease condition. In particular, the ability to distinguish SIRS from sepsis in a patient is highly desirable.

There is therefore an immediate need for the identification of biomarkers that are measurable and specific for the condition, and indicative of the risk of progression to, or early onset of, sepsis as well as methods of using said markers in screening.

Biomarkers are biological indicators that signal a changed physiological state due to a disease or therapeutic intervention. It has been demonstrated that certain substances, including proteins and peptides, are expressed differentially in diseased tissue and bodily fluid samples in certain conditions such as sepsis, when compared to normal tissue and bodily fluid samples. Hence, differentially expressed protein/peptides(s) present in (or absent from) diseased samples from a patient, whilst being absent (or present) in normal tissue, is/are candidate biomarkers for that disease or condition.

Often a single biomarker alone may be insufficient for the accurate diagnosis of a disease or condition, especially one as complex as sepsis. As a result there is a continuing need for identification of biomarkers that may be used to identify or profile the condition at various stages in its pathology.

The only FDA-approved diagnostic biomarker for distinguishing sepsis from non-infectious causes of systemic inflammatory response syndromes (SIRS) currently available is Procalcitonin (PCT). The diagnostic and prognostic performance of PCT is however rather low as was shown in a recent report of Tang and coworkers (Tang B.M.J. et al., The Lancet vol7:p210-217, 2007), indicating that the procalcitonin test cannot accurately distinguish sepsis from SIRS in critically ill adult patients.

C-reactive protein is a further widely used marker for diagnosing sepsis, but is unable to distinguish between sepsis and SIRS without infection.

US 2004/096917 disclose diagnosis of sepsis or SIRS using biomarker profiles.

Roth et al. (Biochemical and biophysical research communications, 2003, vol. 308, no. 4, pages 840-846) disclose the measurement of soluble histones in sera of sepsis patients compared to healthy controls using an ELISA assay against DNA associated histone fragments.

WO 2009/061918 A1 which is an Article 54(3) EPC document disclose that histone proteins are present in higher amounts in the blood of animals suffering from induced sepsis by introduction of lipopolysaccharide (LPS) as compared with control animals.

The inventors have now developed methods that enable rapid quantification, qualification and comparison of protein and peptide profiles derived from different biological samples and as a result have identified novel biomarkers for diagnosis, prognosis and/or prediction of sepsis and its different stages and of sepsis versus SIRS without infection in a subject.

### Summary of the invention

The present invention provides novel biomarkers that enable the medical doctor or the clinician to differentiate between SIRS and sepsis conditions and methods for accurate, rapid, and sensitive prediction, prognosis and/or diagnosis of sepsis versus SIRS through (1) a measurement of the quantity or quality of one or more of said biomarkers taken from a biological sample from a reference subject, be it a healthy subject, a patient having sepsis or a patient having SIRS without infection, to provide a "reference biomarker profile" for said biomarkers that is indicative of the condition and (2) through comparison of this reference biomarker profile with a "candidate biomarker profile" of said biomarker(s) from a comparable biological sample from a subject that has sepsis, is at risk of developing sepsis, or is at a particular stage in the progression of sepsis.

A "reference biomarker profile" may be obtained from a population of individuals who (1) do not have and have never had sepsis, (2) who have sepsis or are suffering from the onset of sepsis or a particular stage in the progression of sepsis or (3) who have SIRS without infection. If the biomarker profile from the test subject contains characteristic features of the biomarker profile from the reference population, then the individual can be diagnosed as respectively being (1) healthy, (2) being at risk of developing sepsis, having sepsis or as being at the particular stage in the progression of sepsis or (3) as having SIRS. The reference biomarker profile may also be obtained from various populations of individuals including those who are suffering from SIRS or those who are suffering from an infection but who are not suffering from SIRS.

Accordingly, the present invention allows the clinician to distinguish between those patients who have SIRS but are not likely to develop severe sepsis, who have sepsis, or who are at risk of developing sepsis.

The present invention provides subject-matter as set forth in any one and all of the appending claims 1 to 10. The specification further provides the use of Histone proteins as defined herein for the prediction, prognosis and/or diagnosis of sepsis or for the differentiation between SIRS and sepsis in a subject.

The specification also provides a method for the prediction, prognosis and/or diagnosis of sepsis or for the differentiation between SIRS and sepsis in a subject comprising obtaining a candidate biomarker profile from a biological sample taken from said subject wherein said candidate biomarker profile is based on the measurement of the quantity of Histone proteins, in said sample and comparing said candidate biomarker profile with a reference biomarker profile obtained form a healthy subject or a patient having SIRS.

Also provided by the specification is a method for the prediction, prognosis and/or diagnosis of sepsis or for the differentiation between SIRS and sepsis in a subject comprising: obtaining a candidate biomarker profile from a biological sample taken from said subject wherein said candidate biomarker profile is based on at least one or two biomarkers selected from the group consisting of Histone proteinspro-Hepcidin (pro-HEPC), soluble TNF-receptor 2 (sTNFR2), Pentraxin-3 (PTX-3), Macrophage Colony-Stimulating Factor (MCSF), pro-Brain Natriuretic Protein (pro-BNP), Procalcitonin (PCT) and c-Reactive Protein (CRP) and comparing said candidate profile with a reference biomarker profile obtained form a healthy subject or a patient having SIRS.

The specification further provides for a method for the prediction, prognosis and/or diagnosis of sepsis or for the differentiation between SIRS and sepsis in a subject comprising measuring the level of at least one or two biomarkers selected from the group consisting of Histone proteins, pro-Hepcidin (pro-HEPC), soluble TNF-receptor 2 (sTNFR2), Pentraxin-3 (PTX-3), Macrophage Colony-Stimulating Factor (MCSF), pro-Brain Natriuretic Protein (pro-BNP), Procalcitonin (PCT) and c-Reactive Protein (CRP) in a biological sample from said subject, using said obtained measurements to create a profile for said biomarkers and comparing said profile with a reference biomarker profile obtained form a healthy subject or a patient having SIRS.

The specification provides for a method for the prediction, prognosis and/or diagnosis of sepsis or for the differentiation between SIRS and sepsis in a subject comprising determining a quantity of at least one or two biomarkers selected from the group consisting of Histone proteins, pro-Hepcidin (pro-HEPC), soluble TNF-receptor 2 (sTNFR2), Pentraxin-3 (PTX-3), Macrophage Colony-Stimulating Factor (MCSF), pro-Brain Natriuretic Protein (pro-BNP), Procalcitonin (PCT) and c-Reactive Protein (CRP) in a sample obtained from a subject; and comparing the quantity of the selected biomarkers in the test subject sample with a range of normal values of the selected biomarkers in control subjects; whereby an increase or decrease in the quantity of the selected biomarker in the sample to a level higher or lower than the range of normal values of the selected biomarkers is indicative of sepsis.

The specification further provides for a method for the prediction, prognosis and/or diagnosis of sepsis or for the differentiation between SIRS and sepsis in a subject comprising determining a quantity of at least one or two biomarkers selected from the group consisting of Histone proteins, pro-Hepcidin (pro-HEPC), soluble TNF-receptor 2 (sTNFR2), Pentraxin-3 (PTX-3), Macrophage Colony-Stimulating Factor (MCSF), pro-Brain Natriuretic Protein (pro-BNP), Procalcitonin (PCT) and c-Reactive Protein (CRP) and comparing the quantity of the selected biomarkers in the test subject sample with a range of values of the selected biomarkers obtained from subjects with sepsis; whereby a comparable quantity of the selected biomarkers in said sample to the range of values of the selected biomarkers in subjects with sepsis is indicative of sepsis.

Alternatively, the specification provides for a method for the prediction, prognosis and/or diagnosis of sepsis or for the differentiation between SIRS and sepsis in a subject comprising obtaining a candidate antibody profile from a biological sample taken from said individual wherein said candidate antibody profile is based on an antibody to at least one or two biomarkers selected from the group consisting of Histone proteins, pro-Hepcidin (pro-HEPC), soluble TNF-receptor 2 (sTNFR2), Pentraxin-3 (PTX-3), Macrophage Colony-Stimulating Factor (MCSF), pro-Brain Natriuretic Protein (pro-BNP), Procalcitonin (PCT) and c-Reactive Protein (CRP) and comparing said candidate antibody profile with a reference antibody profile.

The specification further provides for a method for determining whether a subject is responsive to treatment for sepsis with a substance, comprising the steps of obtaining a candidate biomarker profile from a biological sample taken from said individual wherein said candidate biomarker profile is based on at least one or two biomarkers selected from the group consisting of Histone proteins, pro-Hepcidin (pro-HEPC), soluble TNF-receptor 2 (sTNFR2), Pentraxin-3 (PTX-3), Macrophage Colony-Stimulating Factor (MCSF), pro-Brain Natriuretic Protein (pro-BNP), Procalcitonin (PCT) and c-Reactive Protein (CRP) and comparing said candidate profile with a reference biomarker profile.

For example, one of the selected biomarkers is a member of the family of Histone proteins.

Preferably, said Histone proteins are selected from the group of Histone H1.4, Histone H2A (different isoforms), Histone H2B (different isoforms), Histone H3 (different isoforms), Histone H4 (different isoforms).

Preferred samples to be analysed in the method as described herein are blood or urine, more preferable the sample is serum or plasma.

The method as described herein uses immunoassay technology selected from the group of direct ELISA, indirect ELISA, sandwich ELISA, competitive ELISA, multiplex ELISA, radioimmunoassay, or ELISPOT technologies to establish the biomarker profile. Alternatively, the biomarker profile is established using mass spectrometry analysis methods of the proteins present in said sample.

The specification further describes a kit for the prediction, prognosis and/or diagnosis of sepsis comprising binding molecules to at least one or two biomarkers selected from the group consisting of Histone proteins, pro-Hepcidin (pro-HEPC), soluble TNF-receptor 2 (sTNFR2), Pentraxin-3 (PTX-3), Macrophage Colony-Stimulating Factor (MCSF), pro-Brain Natriuretic Protein (pro-BNP), Procalcitonin (PCT) and c-Reactive Protein (CRP). Such a kit may further comprise a biomarker reference profile or a reference value of the quantity of one or more biomarkers as defined herein, obtained from a healthy subject or a subject having SIRS for comparison of the results.

For example, the kit comprises at least binding molecules that are specific for binding a member of the Histone protein family.

For example, the kit comprises two or more binding molecules that are specific for binding different members of the Histone protein family.

Preferably, said Histone proteins are selected from the group of Histone H1.4, Histone H2A (different isoforms), Histone H2B (different isoforms), Histone H3 (different isoforms), Histone H4 (different isoforms).

Preferred binding molecules as described herein are monoclonal antibodies, polyclonal antibodies, aptamers, photoaptamers, specific interacting proteins, specific interacting small molecules.

The specification encompasses a protein microarray comprising protein fragments of at least two biomarkers selected form the group of Histone proteins, pro-Hepcidin (pro-HEPC), soluble TNF-receptor 2 (sTNFR2), Pentraxin-3 (PTX-3), Macrophage Colony-Stimulating Factor (MCSF), pro-Brain Natriuretic Protein (pro-BNP), Procalcitonin (PCT) and c-Reactive Protein (CRP) coated on a solid phase.

The microarray as described herein comprises protein fragments of at least two members of the Histone proteins family. Preferably, said Histone proteins are selected from the group of Histone H1.4, Histone H2A (different isoforms), Histone H2B (different isoforms), Histone H3 (different isoforms), Histone H4 (different isoforms).

Methods as taught herein further comprise methods in which measurements of any combination of the biomarkers selected from the group of Histone proteins, pro-Hepcidin (pro-HEPC), soluble TNF-receptor 2 (sTNFR2), Pentraxin-3 (PTX-3), Macrophage Colony-Stimulating Factor (MCSF), pro-Brain Natriuretic Protein (pro-BNP), Procalcitonin (PCT) and c-Reactive Protein (CRP) are included in the creation of the candidate and reference profile. It will be understood that additional biomarkers may also be included such as biomarkers already used for the diagnosis or prognosis of sepsis or SIRS.

The specification further provides methods as outlined above wherein the profile is created using antibodies to said biomarkers. In this case the candidate and reference biomarker profiles will be created based on measurements of antibodies to the biomarkers and are referred to hereinafter as candidate antibody profiles and reference antibody profiles.

### Brief description of the drawings

Figure 1 illustrates the results of an ELISA experiment detecting the protein level of biomarker X1, sTNFR2 in samples from healthy subjects, samples from patients having SIRS and from patients having sepsis.
Figure 2 illustrates the results of an ELISA experiment detecting the protein level of biomarker X2, PTX-3 in samples from healthy subjects, samples from patients having SIRS and from patients having sepsis.
Figure 3 illustrates the results of an ELISA experiment detecting the protein level of biomarker X3, M-CSF in samples from healthy subjects, samples from patients having SIRS and from patients having sepsis.
Figure 4 illustrates the results of an ELISA experiment detecting the protein level of biomarker X4, pro-HEPC in samples from healthy subjects, samples from patients having SIRS and from patients having sepsis.
Figure 5 illustrates illustrates the results of an ELISA experiment detecting the protein level of biomarker X5, pro-BNP in samples from healthy subjects, samples from patients having SIRS and from patients having sepsis.
Figure 6 shows the predictive value for discriminating between SIRS and sepsis using the PCT marker alone.
Figure 7 shows the predictive value for discriminating between SIRS and sepsis using the CRP marker alone.
Figure 8 shows the predictive value for discriminating between SIRS and sepsis using the sTNFR2 marker alone.
Figure 9 shows the predictive value for discriminating between SIRS and sepsis using the M-CSF marker alone.
Figure 10 shows the predictive value for discriminating between SIRS and sepsis using the pro-HEPC marker alone.
Figure 11 shows the predictive value for discriminating between SIRS and sepsis using the pro-BNP marker alone.
Figure 12 shows the predictive value for discriminating between SIRS and sepsis using the PTX-3 marker alone.
Figure 13 shows the predictive value for discriminating between SIRS and sepsis using a combination of the PTX-3 and pro-HEPC markers.
Figure 14 shows the correlation of the predictive value for discriminating between SIRS and sepsis between the PTX-3 marker and the PCT marker.

### Detailed description of the invention

Sepsis may be characterised as an initial systemic inflammatory response syndrome (SIRS), sepsis, severe sepsis (sepsis with acute organ dysfunction), septic shock (sepsis with refractory arterial hypotension), multiple organ dysfunction or failure and death.

"SIRS" is a systemic inflammatory response syndrome with no signs of infection. It can be characterized by the presence of at least two of the four following clinical criteria: fever or hypothermia (temperature 100,4°F [38°C] or 96,8°F [36°C]), tachycardia (90 beats per minute), tachypnea (20 breaths per minute or PaCO2 4,3 kPa [32 mm Hg] or the need for mechanical ventilation), and an altered white blood cell count of 12,000 cells/ mL, 4000 cells/mL, or the presence of 10% band forms, respectively.

"Sepsis" can be defined as SIRS with an infection. Infection can be diagnosed by standard textbook criteria or, in case of uncertainty, by an infectious disease specialist.

"Severe sepsis" can be defined as the presence of sepsis and at least one of the following manifestations of inadequate organ perfusion or function: hypoxemia (PaO2 10 kPa [75 mm Hg]), metabolic acidosis (pH 7,30), oliguria (output 30 mL/hr), lactic acidosis (serum lactate level 2 mmol/L), or an acute alteration in mental status without sedation (i.e., a reduction by at least 3 points from baseline value in the Glasgow Coma Score).

"Septic shock" can be defined as the presence of sepsis accompanied by a sustained decrease in systolic blood pressure (90 mm Hg, or a drop of 40 mm Hg from baseline systolic blood pressure) despite fluid resuscitation, and the need for vasoactive amines to maintain adequate blood pressure.

As many organisms can be the cause of sepsis, diagnosis often takes time and requires testing against panels of possible agents. Sepsis can also arise in many different circumstances and therefore sepsis can be further classified for example: incarcerated sepsis which is an infection that is latent after the primary lesion has apparently healed but may be activated by a slight trauma; catheter sepsis which is sepsis occurring as a complication of intravenous catheterization; oral sepsis which is a disease condition in the mouth or adjacent parts which may affect the general health through the dissemination of toxins; puerperal sepsis which is infection of the female genital tract following childbirth, abortion, or miscarriage; sepsis len¢ta which is a condition produced by infection with a-hemolytic streptococci, characterized by a febrile illness with endocarditis.

For the purposes of this invention, the wording "sepsis" is used hereafter to include all conditions and stages of the disease progression.

As described herein, sepsis may be predicted or diagnosed by obtaining a profile of biomarkers from a sample obtained from an individual. The methods and kits as taught herein are particularly useful in predicting and diagnosing sepsis in an individual who has an infection, or has sepsis, but who has not yet been diagnosed as having sepsis, who is suspected of having sepsis, or who is at risk of developing sepsis. The methods and kits as taught herein may also be used to differentiate between SIRS and sepsis and to detect and diagnose SIRS in an individual or to detect that a person is not at risk of developing sepsis. The methods and kits as taught herein also may be used to detect various stages of the sepsis progression such as sepsis, severe sepsis, septic shock, and organ failure.

Biomarker profiles may be created in a number of ways and may be a ratio of two or more measurable aspects of a biomarker. A biomarker profile comprises at least two measurements, where the measurements can correspond to the same or different biomarkers. A biomarker profile may also comprise at least three, four, five, 10, 20, 30 or more measurements. A biomarker profile may comprise hundreds, or even thousands, of measurements.

The profile of a biomarkers obtained from an individual namely the candidate biomarker profile, is compared to a reference biomarker profile. The reference biomarker profile can be generated from one individual or a population of individuals. The population, for example, may comprise two, ten, or many more, possibly hundreds of individuals.

The reference biomarker profile and the candidate biomarker profiles that are compared in the methods as taught herein may be generated from the same individual for the purposes on monitoring disease progression. In this instance it would be expected that the candidate and reference profiles are generated from biological samples taken at different time points and compared to one another. Such a comparison may be used, for example, to determine the status of sepsis in the individual by repeated measurements over time.

The reference biomarker profiles may be chosen from individuals who are sepsispositive and suffering from one of the stages in the progression of sepsis, or from individuals with increased risk of developing sepsis, or from populations of individuals who do not have SIRS, from individuals who do not have SIRS but who are suffering from an infectious process, from individuals who are suffering from SIRS without the presence of sepsis or from individuals who are suffering from the onset of sepsis. The reference biomarker profile may be generated from a healthy population.

The methods as taught herein comprise comparing a candidate biomarker profile with a reference biomarker profile. As used herein, comparison includes any means to determine at least one difference in the candidate and the reference biomarker profiles. A comparison may include a visual inspection, an arithmetical or statistical comparison of measurements. Such statistical comparisons include, but are not limited to, applying a rule. If the biomarker profiles comprise at least one standard, the comparison to determine a difference in the biomarker profiles may also include measurements of these standards, such that measurements of the biomarker are correlated to measurements of the internal standards. The comparison should enable prognosis, diagnosis and/or predication of the presence of sepsis of increased risk of sepsis of, SIRS, or even absence of sepsis or SIRS. Alternatively, the comparison can indicate the stage of sepsis at which an individual may be.

The methods and kits as taught herein are based on the identification of six new biomarkers of sepsis. However, these may be used in conjunction with other biomarkers and these may include any biological compound such as a protein or fragment thereof, a peptide, a polypeptide, a proteoglycan, a glycoprotein, a lipoprotein, a carbohydrate, a lipid, a nucleic acid, or other polymer, or any biological molecule that is present in the biological sample and that may be isolated from, or measured in, the biological sample. Furthermore, a biomarker can be the entire molecule, or it can be part thereof that may be partially functional or recognized, for example, by an antibody, aptamer or other specific binding molecule. A biomarker is useful if it is specific for sepsis and measurable. Such a measurable aspect may include, for example, the presence, absence, or concentration of the biomarker in the biological sample from the individual and/or its presence as part of a profile of biomarkers.

### Biomarkers identified

As is clear from the examples below, we analysed blood samples from subjects suffering from SIRS, subjects suffering from sepsis in several stages of severity and form healthy subjects. The study was run in reference design mode on Pronota's MASStermind discovery platform, using the well known Cofradic procedure. In a reference design study each sample is measured against a reference sample, typically a pool of all patient samples. For each feature present in a certain sample a ratio is obtained which represents the fold difference of the feature intensity in the reference versus feature intensity in the sample. Combining all feature data from all samples into an expression matrix allows comparing features intensities between samples and between groups of samples.

For the SIRS/SEPSIS a 10-10 reference design study was set up and hence the reference pool was a pool of all 20 samples. An intermediate matrix analysis was performed after 3 samples of each group were run through the platform.

All serum samples were delipidated followed by depletion for the most abundant proteins using a Beckman Ig-Y12 column. Depletion efficiency was checked using ELISAs and Western Blot analysis. The reference pool was prepared at this stage and this reference was considered as a normal sample for the rest of process. Samples were prepared for MASStermind analysis according the standard N-ter COFRADIC procedures. Reference pool and samples were differentially labeled by trypsin mediated incorporation of ¹⁸O/¹⁶O, where the different samples carried the heavy oxygen label and the reference the ¹⁶O. Just before COFRADIC sorting each sample was mixed with the reference at equal protein masses. After sorting, NanoLC separations MS spectra were obtained. MS data were deisotoped, clustered and features were constructed using in house developed software called euCatLabel. The output of this data processing is an expression matrix containing all features from all analyzed samples. Each feature is represented by a unique combination of m/z, COFRADIC sorting pool and NanoLC retention time and features can be present in a number of samples ranging from 1 to all. If a feature is present in a sample it will carry a ratio, which represents intensity of the feature in the reference sample (ie the ¹⁶O peak) over the intensity in the respective sample (the ¹⁸O peak). Recurrent quantifiable features are features with a reliable ratio reading in at least one sample. For the intermittent matrix analysis of 3 samples each, all features were considered that were either present in 5/6 samples or were present in all 3 samples of the SIRS patient group and in none of the samples of the SEPSIS patient group.

Using this approach, we identified six biomarkers that were present in differing quantities in samples obtained from SIRS patients and sepsis patients. These markers are listed in Table 1 below. In order to assess the use of these biomarkers in the diagnosis of sepsis and in the differentiation between SIRS and sepsis, several ELISA measurements were performed and the date was used to assess the power of the biomarkers or combinations thereof in differentiating between SIRS and sepsis in a subject. The six identified biomarkers are described in some further detail below.

| **Table 1: newly identified SIRS / sepsis biomarkers** | | | |
|---|---|---|---|
| **ID** | **Name** | | **Genbank Acc. No.** |
| X1 | sTNFR2 | Soluble TNF Receptor 2 | NP_001057 |
| X2 | PTX-3 | Pentraxin-3 | NP_002843 |
| X3 | M-CSF | Macrophage Colony Stimulating Factor | NP_000748 |
| X4 | Pro-HEPC | Hepcidin pro form | NP_066998 |
| X5 | Pro-BNP | Brain Natriuretic Protein pro form | NP_002512 |
| X6 | Histones | Cf. Table 1b | |

### X1: soluble TNF-receptor 2 (sTNFR2)

Soluble TNF-receptor 2 (TNF-sr75 or sTNFR2) has been shown to be a putative marker for diagnosing sepsis by Neilson et al., 1996 (Cytokine. 1996 vol.12:938-43). The levels of TNF-alpha and TNF-soluble receptor were raised in patients who became clinically septic and correlated well with the severity of sepsis (using the APACHE III score). In septic patients there was no difference in the pattern of changes in the two types of receptor (TNF-sr55 and TNF-sr75). However, in non-septic patients TNF-sr75 was higher in those with endotoxaemia than those without. This difference was not observed with TNF-sr55 which suggests a different mechanism of release or degree of sensitivity for the two soluble receptors. Regardless of severity of illness, the levels of all three molecules (TNF-alpha and the two receptors) appeared to start rising at about the same time point. The peak TNF-alpha level was reached earlier (2-4 h) than that of the two TNF-sr (4-8 h). The relative rise in TNF-alpha was greater than that of the soluble receptors and this difference was even more marked in those with more severe sepsis. The relationship between peak TNF-alpha and peak TNF-sr was non-linear and the concentration of each TNF-sr appeared to plateau at the higher levels of TNF-alpha. This suggests the exhaustion of a limited pool or saturation of the rate of release. Taken together, these results suggest sepsis develops because of delayed and insufficient secretion of TNF-sr compared with TNF-alpha.

### X2: Pentraxin 3 (PTX-3)

The Pentraxins are highly conserved proteins throughout evolution starting from *the* horse shoe crab to man. The classic pentraxins are of the short form and are known as C-reactive protein (CRP) and serum amyloid P component (SAP). They are made in the liver upon stimulus from interleukin (IL)-6. Measurement of CRP is routinely used for diagnosis and monitoring of diverse inflammatory and infectious diseases, whereas SAP is a useful probe to identify amyloid deposits. Pentraxin 3 (PTX3 or TNF-stimulated gene 14 (TSG-14)) is the first identified so called long pentraxin, consisting of a C-terminal pentraxin module coupled with an unrelated N-terminal domain. It differs from the classic short pentraxins in terms of gene organization and localization, ligand recognition, producing cells, and inducing signals. PTX3 is made in response to primary proinflammatory signals (bacterial products, IL-1, and tumor necrosis factor [TNF] but not IL-6) by diverse cell types, predominantly macrophages and endothelial cells. As was demonstrated by Muller et al., (Crit Care Med 2001 Vol. 29, No. 7), PTX3 is elevated in patients with SIRS, sepsis, and septic shock. Maximal elevations of PTX3 were observed in patients with septic shock. Furthermore, PTX3 levels were correlated with disease severity as assessed by clinical scores. In particular, a dramatic difference was apparent in PTX3 levels at admission or on day 2 between patients who survived and those who eventually died.

### X3: Macrophage Colony Stimulating Factor (M-CSF)

M-CSF is a hematopoietic growth factor that mainly stimulates the growth, differentiation, and proliferation of cells of the monocyte-macrophage lineage. Ören et al., 2001 (Pediatrics, 2001 vol.108:329-332) studied the serum M-CSF levels in healthy, septic, and hypoxic term neonates on the first day of life and examined the relationship of serum M-CSF levels and circulating monocyte and thrombocyte counts in these newborn infants. Three groups were defined in this prospective study: group 1, healthy neonates with no risk factors (*n* 5 40); group 2, neonates who had severe hypoxia (*n* 5 20); and group 3, neonates who fulfilled the criteria for early-onset sepsis (*n* 5 18). Blood samples were collected for complete blood cell count and serum M-CSF levels by peripheral venipuncture from each infant in the first 24 hours after birth before any medical therapy. The gestational ages and birth weights did not differ significantly between the groups. Serum MCSF levels of the septic neonates were significantly higher than of both healthy and hypoxic neonates, but did not differ significantly between the healthy and hypoxic neonates. There was no significant correlation between serum M-CSF levels and circulating monocyte counts, but there was a significant inverse correlation between serum M-CSF levels and thrombocyte counts. When this correlation was analyzed according to groups, it was determined that this inverse correlation between MCSF levels and thrombocyte counts was especially significant in the septic neonate group, but not significant in the healthy and hypoxic neonate groups. Serum M-CSF levels are significantly higher in neonates with sepsis. High serum M-CSF levels may have a possible role in the pathogenesis of thrombocytopenia in neonates with sepsis.

### X4: pro-Hepcidin (pro-HEPC)

Hepcidin (cf. Malyszko and Mysliwiec, Kidney Blood Press Res 2007;30:15-30 for an overview) is a circulating antimicrobial peptide mainly synthesized in the liver, which has been recently proposed as a factor regulating the uptake of dietary iron and its mobilization from macrophages and hepatic stores. Inflammation causes an increase of production of hepcidin, which is a potent mediator of anemia of chronic diseases. Hepcidin *(hep* atic bacteri *cid* al prote *in*), a recently discovered small, cysteine-rich cationic peptide from plasma, produced by the liver and had antimicrobial properties.

The structure of hepcidin is highly conservative among mammals, suggesting a key role in major biological functions. Closely related hepcidin sequences are found in vertebrates from fish to humans.

After a cleavage of the 24 amino acid N-terminal signal peptide, prohepcidin is transported through the hepatocyte basolateral membrane into the circulation. The major circulating bioactive forms of hepcidin consist only of the carboxy-terminal portion (peptides of 25, 22 and 20 amino acids). The exact location of the final prohormone processing is unknown. Propeptide convertases could be located in the blood or in the cell membrane of capillaries. There is still no reliable information available on normal serum levels of mature hepcidin. Substantial progress has been made to elucidate the mechanism of action of hepcidin. Presently, the main hepcidin function is homeostatic regulation of iron metabolism and mediation of host defense and inflammation. The hepcidin gene is overexpressed in livers of experimentally iron-loaded mice and hepcidin knockout mice develop iron overload. Injection of hepcidin inhibits intestinal iron absorption in mice independent of their iron status. It was shown that hepcidin injection results in a dose-dependent in serum iron. Conversely, iron ingestion in humans induced hepcidin secretion in urine. These observations suggest that hepcidin plays a role as a negative regulator of intestinal iron absorption and iron release from macrophages. Evidence from transgenic mouse models indicates that hepcidin is the predominant negative regulator of iron absorption in the small intestine, iron transport across the placenta, and iron release from the macrophages. Hepcidin controls intestinal iron absorption by regulating ferroportin expression on the basolateral membrane of enterocytes. Hepcidin directly regulates the expression of the ferroportin on cell membranes, acts by binding to the cellular iron exporter ferroportin and inducing its internalization and degradation, thus trapping iron in enterocytes, macrophages and hepatocytes. The net effect of hepcidin is the diminished absorption of dietary iron, sequestration of iron in macrophages and sequestration of iron in hepatic stores. The absence of hepcidin synthesis would naturally lead to a major loss of control over iron release by enterocytes and macrophages followed by circulatory iron overload. Hemojuvelin is probably a key modulator of hepcidin expression. Hemojuvelin generates bone morphogenic protein, which increases hepcidin mRNA levels. Hepcidin is also known as liver-expressed antimicrobial peptide-1 (LEAP-1). No correlation with diagnosis of sepsis or SIRS has been established.

### X5: Brain Natriuretic Protein (pro-BNP)

Brain natriuretic peptide (also known as *B-type natriuretic peptide* or "GC-B") is a 32 amino acid polypeptide secreted by the ventricles of the heart in response to excessive stretching of myocytes (heart muscles cells) in the ventricles. At the time of release, a co-secreted 76 amino acid N-terminal fragment (NT-proBNP) is also released with BNP. BNP binds to and activates NPRA in a similar fashion to atrial natriuretic peptide (ANP) but with 10-fold lower affinity. The biological half-life of BNP, however, is twice as long as that of ANP. Both ANP and BNP have limited ability to bind and activate NPRB.

Brain natriuretic peptide was originally identified in extracts of porcine brain, but in humans it is produced mainly in the cardiac ventricles.

Physiologic actions of BNP and ANP include decrease in systemic vascular resistance and central venous pressure as well as an increase in natriuresis. Thus, the resulting effect of these peptides is a decrease in cardiac output and a decrease in blood volume.

Little is known about the utility of natriuretic peptides for reflecting left ventricular dysfunction and predicting survival in septic patients. In the context of severe sepsis, an analysis by Brueckmann and coworkers (Circulation 2005, Vol112:527-534) showed that BNP may be useful as a marker to predict survival in patients presenting with severe sepsis.

### X6: Histones

The final protein we picked up in this analysis was identified as a member of the histone family. Although it is at present unclear why histone proteins would be present in higher amounts in blood samples of patients having sepsis as compared to SIRS or healthy subjects, we think it could be due to a recently discovered mechanism of innate host defense called Neutrophil Extracellullar Traps or NETs (cf. Brinkmann and Zychlinsky, Nature Reviews Microbiology, 2007 vol 5:1-6). Neutrophils are one of the main types of effector cell in the innate immune system and are known to effectively kill microorganisms by phagocytosis. Recently, however, it has been found that stimulated neutrophils can also produce NETs that capture and kill microorganisms. The backbone of the NETs constitutes out of chromatin released from the suicidal neutrophil and take the form of DNA-based threads and cables. In addition to DNA, some proteins, such as histones appear to form some globular domains in these NETs. Microorganisms are killed by neutrophil cultures that have been stimulated to produce NETs by IL-8, bacterial components, or the protein kinase C activator PMA, and in which phagocytosis is prevented, indicating that NETs have antimicrobial activity. Furthermore, this activity can be eliminated if the NETs are disrupted by treating activated neutrophils with DNases. After an infection, neutrophils will first kill bacteria by phagocytosis and only later by the production of NETs. How neutrophils become committed to NET formation is not yet understood, but both antimicrobial mechanisms are equally effective. The high local concentration of antimicrobial agents in NETs is essential for bacterial killing. Interestingly, almost all bacteria analyzed so far appear to be sensitive towards histone 2A, which is one of the most effective antimicrobial agents. Several investigators identified histones and histone fragments from diverse sources as having antimicrobial activity. At that time, the biological significance of this observation was unclear, as it was not understood where histones would encounter microorganisms during an infection. This can now be explained with the discovery of NETs. Interestingly, NETs kill pathogenic fungi in both their yeast and hyphal forms. However, histones are not involved in the killing of these eukaryotes and this indicates that NETs might contain specific antifungal agents. The specific contribution of the granular antimicrobial peptides, proteases and other enzymes or the histones to microbial killing is not understood. It is possible that different pathogens have different levels of susceptibility to these components. NETs could also make microorganisms susceptible to other antimicrobial effectors.

Sepsis is caused by an infection of a microorganism, and it has been demonstrated that the innate immune system is activated in patients at the onset of sepsis. This response comprises of several events, one of them being the production of NETs in the blood, comprised of chromatin back-bone, coated with intrecallular and intrenuclear proteins such as histones. This might in fact be the explanation of the increase of histone protein in the blood of patients with sepsis. In samples from patients with SIRS, no such histone presence in the blood was detected. This means that histones as identified by the method as described herein may be a very clear indicator of sepsis enabling the differentiation between SIRS and sepsis patients.

The next table summarizes all histone modified peptide sequences. Some of these are unique for a certain histone subtype while other match to different histone type isoforms. We do detect all major histone classes (1-4).

**Table 1b: Identified histone proteins**

| **Protein** | **Modified peptide sequence** | **position** |
|---|---|---|
| Histone H1.4 | <Acetyl (N-term)>SETAPAAPAAPAPAEKTPVKKKAR | 1-24 |
| **Histone H2A** (different isoforms) | AGLQFPVGR | 21-29 |
| Histone H2B (different isoforms) | <Acetyl (N-term)>AVTKAQKKDGKKR | 17-29 |
| Histone H3 (different isoforms) | <Acetyl (N-term)>SAPATGGVKKPHR | 28-40 |
| Histone H3 (different isoforms) | <Acetyl (N-term)>YQKSTELLIR | 54-63 |
| Histone H3 (different isoforms) | <Pyro-glu (N-term E)>EIAQDFKTDLR | 73-83 |
| Histone H3 (different isoforms) | <Acetyl (N-term)>KSTGGKAPR | 9-17 |
| Histone H4 | DNIQGITKPAIR | 24-35 |

Any of the above markers identified herein (X1-X6) can be used separately or in combination in the kits, microarrays and methods as described herein. Any combination of two or more of the markers identified herein can be used together. In addition, any combination of one or more of the newly identified biomarkers can be used together with other known sepsis markers. One preferred known sepsis marker is the PCT (procalcitonin) marker or the CRP (c-reactive protein) marker. Preferred combinations of the markers are the following:
Pro-BNP + PTX-3; pro-BNP + pro-HEPC; pro-BNP + sTNFR2; pro-BNP + M-CSF; pro-BNP + Histone proteins; PTX-3 + pro-HEPC; PTX-3 + sTNFR2; PTX-3 + M-CSF; PTX-3 + Histone proteins; pro-HEPC + sTNFR2; pro-HEPC + M-CSF; pro-HEPC + Histone proteins; M-CSF + sTNFR2; M-CSF + Histone proteins; sTNFR2 + Histone proteins and any of these combinations + PCT or CRP.

Pro-BNP + PTX-3 + pro-HEPC; Pro-BNP + PTX-3 + sTNFR2; Pro-BNP + PTX-3 + M-CSF; pro-BNP + pro-HEPC + sTNFR2; pro-BNP + pro-HEPC + M-CSF; pro-BNP + sTNFR2 + M-CSF; PTX-3 + pro-HEPC + sTNFR2; PTX-3 + pro-HEPC + M-CSF; PTX-3 + sTNFR2 + M-CSF; pro-HEPC + sTNFR2 + M-CSF; Histone proteins + Pro-BNP + PTX-3; Histone proteins + Pro-BNP + pro-HEPC; Histone proteins + Pro-BNP + sTNFR2; Histone proteins + Pro-BNP + M-CSF; Histone proteins + PTX-3 + pro-HEPC; Histone proteins + PTX-3 + sTNFR2; Histone proteins + PTX-3 + M-CSF; Histone proteins + pro-HEPC + sTNFR2; Histone proteins + pro-HEPC + M-CSF; Histone proteins + M-CSF + sTNFR2 and any of these combinations + PCT or CRP.

For example, the combination always comprises one or more members of the Histone protein family as a biomarker in combination with any one or more other biomarkers of sepsis or SIRS

The most preferred combinations are PTX-3 + pro-HEPC; and PCT + pro-HEPC + sTNFR2.

### Procalcitonin (PCT)

Procalcitonin (PCT) is a precursor of the hormone calcitonin, which is involved with calcium homeostasis, and is produced by the C-cells of the thyroid gland. It is there that procalcitonin is cleaved into calcitonin, katacalcin and a protein residue. It is not released into the blood stream of healthy individuals. With the derangements that a severe infection with an associated systemic response brings, the blood levels of procalcitonin may rise to 100 ng/ml. In blood serum, procalcitonin has a half-life of 25 to 30 hours. Measurement of procalcitonin can be used as a marker of severe sepsis and generally grades well with the degree of sepsis, although levels of procalcitonin in the blood are very low. PCT has the greatest sensitivity (85%) and specificity (91%) for differentiating patients with SIRS from those with sepsis, when compared with IL-2, IL-6, IL-8, CRP and TNF-alpha. However, the test is not routinely used and has yet to gain widespread acceptance (cf. Meisner et al., 1999, Crit Care vol3(1):45-50).

### C-reactive protein (CRP)

C-reactive protein (CRP) is a plasma protein, an acute phase protein produced by the liver and by adipocytes. It is a member of the pentraxin family of proteins and has been widely used as a marker for sepsis, especially in neonates. Its accuracy is however controversial.

### Generation of biomarker profiles

Biomarker profiles may be generated by the use of one or more separation methods. For example, suitable separation methods may include a mass spectrometry method, such as electrospray ionization mass spectrometry (ESI-MS), ESI-MS/MS, ESI-MS/ (MS)" (n is an integer greater than zero), matrix-assisted laser desorption ionization time- of-flight mass spectrometry (MALDI-TOF-MS), surface-enhanced laser desorption/ionization time-of-flight mass spectrometry (SELDI-TOF-MS),desorption/ionization on silicon (DIOS), secondary ion mass spectrometry (SIMS), quadrupole time-of-flight (Q-TOF), atmospheric pressure chemical ionization mass spectrometry (APCI-MS), APCI-MS/MS, APCI- (MS)", atmospheric pressure photoionization mass spectrometry (APPI-MS),APPI-MS/MS, and APPI- (MS)". Other mass spectrometry methods may include, inter alia, quadrupole, fourier transform mass spectrometry (FTMS) and ion trap. Other suitable separation methods may include chemical extraction partitioning, column chromatography, ion exchange chromatography, hydrophobic (reverse phase) liquid chromatography, isoelectric focusing, one-dimensional polyacrylamide gel electrophoresis (PAGE), two-dimensional polyacrylamide gel electrophoresis (2D-PAGE) or other chromatography, such as thin-layer, gas or liquid chromatography, or any combination thereof. The biological sample may be fractionated prior to application of the separation method.

Biomarker profiles may also be generated by methods that do not require physical separation of the biomarkers themselves. For example, nuclear magnetic resonance (NMR) spectroscopy may be used to resolve a profile of biomarkers from a complex mixture of molecules. An analogous use of NMR to classify tumors is disclosed in Hagberg, NMR Biomed. 11: 148-56 (1998), for example. Additional procedures include nucleic acid amplification technologies, which may be used to generate a profile of biomarkers without physical separation of individual biomarkers. (See Stordeur et al., J. Immunol. Methods 259: 55-64 (2002) and Tan et al., Proc. Nat. Acad. Sci. USA 99: 11387-11392 (2002), for example). Laser desorption/ionization time-of-flight mass spectrometry may be used to create a profile of biomarkers where the biomarkers are proteins or protein fragments that have been ionized and vaporized off an immobilizing support by incident laser radiation. A profile is then created by the characteristic time-of-flight for each protein, which depends on its mass-to-charge ("m/z") ratio. A variety of laser desorption/ionization techniques are known in the art. (See, e.g., Guttman et al., Anal. Chem. 73: 1252-62 (2001) and Wei et al., Nature 399 : 243-46 (1999)). Laser desorption/ionization time-of-flight mass spectrometry allows the generation of large amounts of information in a relatively short period of time. A biological sample is applied to one of several varieties of a support that binds all of the biomarkers, or a subset thereof, in the sample. Cell lysates or samples are directly applied to these surfaces in volumes as small as 0.5RL, with or without prior purification or fractionation. The lysates or sample can be concentrated or diluted prior to application onto the support surface. Laser desorption/ionization is then used to generate mass spectra of the sample, or samples, in as little as three hours.

The protein biomarker profile as described herein may be established using immunoassay technologies such as direct ELISA, indirect ELISA, sandwich ELISA, competitive ELISA, multiplex ELISA, radioimmunoassay, ELISPOT technologies, and other similar techniques known in the art.

The direct ELISA uses the method of directly labeling the antibody itself. Microwell plates are coated with a sample containing the target antigen, and the binding of labeled antibody is quantitated by a colorimetric, chemiluminescent, or fluorescent end-point. Since the secondary antibody step is omitted, the direct ELISA is relatively quick, and avoids potential problems of cross-reactivity of the secondary antibody with components in the antigen sample. However, the direct ELISA requires the labeling of every antibody to be used, which can be a time-consuming and expensive proposition. In addition, certain antibodies may be unsuitable for direct labeling. Direct methods also lack the additional signal amplification that can be achieved with the use of a secondary antibody.

The indirect, two-step ELISA method uses a labeled secondary antibody for detection. First, a primary antibody is incubated with the antigen. This is followed by incubation with a labeled secondary antibody that recognizes the primary antibody. For ELISA it is important that the antibody enzyme conjugate is of high specific activity. This is achieved when the antibody is affinity purified and the enzyme conjugation chemistry preserves antibody specificity as well as enzyme activity

The sandwich ELISA measures the amount of antigen between two layers of antibodies. The antigens to be measured must contain at least two antigenic sites, capable of binding to the antibody, since at least two antibodies act in the sandwich. For this reason, sandwich assays are restricted to the quantitation of multivalent antigens such as proteins or polysaccharides. Sandwich ELISAs for quantitation of antigens are especially valuable when the concentration of antigens is low and/or they are contained in high concentrations of contaminating protein. To utilize this assay, one antibody (the "capture" antibody) is purified and bound to a solid phase typically attached to the bottom of a plate well. Antigen is then added and allowed to complex with the bound antibody. Unbound products are then removed with a wash, and a labeled second antibody (the "detection" antibody) is allowed to bind to the antigen, thus completing the "sandwich". The assay is then quantitated by measuring the amount of labeled second antibody bound to the matrix, through the use of a colorimetric substrate. Major advantages of this technique are that the antigen does not need to be purified prior to use, and that these assays are very specific. However, one disadvantage is that not all antibodies can be used. Monoclonal antibody combinations must be qualified as "matched pairs", meaning that they can recognize separate epitopes on the antigen so they do not hinder each other's binding. The ELISA kits are good enough to reach detection senility at sub-nanogram per ml level and are useful for screening protein targets and quantifying their expression in different conditions. For higher detection sensitivity needed, monoclonal antibodies can be further introduced into the ELISA kit to pair with polyclonal IgY as either capture or detection antibodies.

When two "matched pair" antibodies are not available for a target, another option is the competitive ELISA. The advantage to the competitive ELISA is that non-purified primary antibodies may be used. Although there are several different configurations for competitive ELISA, one reagent must be conjugated to a detection enzyme, such as horseradish peroxidase. The enzyme may be linked to either the antigen or the primary antibody. An example of such a competitor is a labeled antigen. In this type of ELISA, there is an inverse relationship between the signal obtained and the concentration of the analyte in the sample, due to the competition between the free analyte and the ligand-enzyme conjugate for the antibody coating the microplate, i.e. the more analyte the lower the signal. Briefly, an unlabeled purified primary antibody is coated onto the wells of a 96 well microtiter plate. This primary antibody is then incubated with unlabeled standards and unknowns. After this reaction is allowed to go to equilibrium, conjugated antigen is added. This conjugate will bind to the primary antibody wherever its binding sites are not already occupied by unlabeled antigen. Thus, the more unlabeled antigens in the sample or standard, the lower the amount of conjugated antigen bound. The plate is then developed with substrate and color change is measured.

Multiplex ELISA is a microtiter plate ELISA-based protein array assay that allows simultaneous detection of multiple analytes at multiple array addresses within a single well. There are different types of multiplex ELISA have been developed and in practice. One of the examples is to measure antigens by coating or printing capture antibodies in an array format within a single well to allow for the construction of "sandwich" ELISA quantification assays. Generally, multiplex ELISA can also be achieved through antibody array, where different primary antibodies can be attached to a solid phase e.g. a glass plate to capture corresponding antigens in a biological sample. The detection method can be direct or indirect, sandwich or competitive, labeling or non-labeling, depending upon antibody array technologies.

The Enzyme-Linked Immunosorbent Spot (ELISpot) assay employs the sandwich assay approach of the Enzyme-Linked ImmunoSorbent Assay (ELISA), with some variations. The capture antibody is coated aseptically onto a polyvinylidene difluoride (PVDF)-backed microwell plate. The plate is blocked with serum proteins, cells of interest are plated out at varying densities, along with antigen or mitogen, and plates are incubated at 37°C. Cytokine secreted by activated cells is captured locally by the coated antibody on the high surface area PVDF membrane. The wells are washed to remove cells, debris, and media components. A second antibody (biotinylated) reactive with a distinct epitope of the target cytokine is employed to detect the captured cytokine. The detected cytokine is then visualized using avidin-HRP, and a precipitating substrate (e.g. AEC). The colored end product (spot) represents an individual cytokine-producing cell. The spots can be counted manually (e.g., with a dissecting microscope) or using an automated reader to capture the microwell images and to analyze spot number and size.

Radioimmunoassay (RIA) involves mixing known quantities of radioactive antigen (frequently labeled with gamma-radioactive isotopes of iodine attached to tyrosine) with antibody to that antigen, then adding unlabeled or "cold" antigen and measuring the amount of labeled antigen displaced. Initially, the radioactive antigen is bound to the antibodies. When "cold" (unlabeled, quest) antigen is added, the two compete for antibody binding sites - at higher concentrations of "cold" antigen, more of it binds to the antibody, displacing the radioactive variant. The bound antigens are separated from the unbound ones.

As used herein, the term "profile" includes any set of data that represents the distinctive features or characteristics associated with a condition of sepsis. The term encompasses a "nucleic acid profile" that analyzes one or more genetic markers, a "protein profile" that analyzes one or more biochemical or serological markers, and combinations thereof. Examples of nucleic acid profiles include, but are not limited to, a genotypic profile, gene copy number profile, gene expression profile, DNA methylation profile, and combinations thereof. Non-limiting examples of protein profiles include a protein expression profile, protein activation profile, protein cleavage profile, and combinations thereof. For example, a "genotypic profile" includes a set of genotypic data that represents the genotype of one or more genes associated with a condition of sepsis. Similarly, a "gene copy number profile" includes a set of gene copy number data that represents the amplification of one or more genes associated with a condition of sepsis. Likewise, a "gene expression profile" includes a set of gene expression data that represents the mRNA levels of one or more genes associated with a condition of sepsis. In addition, a "DNA methylation profile" includes a set of methylation data that represents the DNA methylation levels (e.g., methylation status) of one or more genes associated with a condition of sepsis. Furthermore, a "protein expression profile" includes a set of protein expression data that represents the levels of one or more proteins associated with a condition of sepsis. Moreover, a "protein activation profile" includes a set of data that represents the activation (e.g., phosphorylation status) of one or more proteins associated with a condition of sepsis. A "protein cleavage profile" includes a set of data that represent the proteolytic cleavage of one or more proteins associated with a condition of sepsis.

The term "subject" or "patient" typically includes humans, but can also include other animals such as, e.g., other primates, rodents, canines, felines, equines, ovines, porcines, and the like.

The term "sample" as used herein includes any biological specimen obtained from a subject. Samples include, without limitation, whole blood, plasma, serum, red blood cells, white blood cells (e.g., peripheral blood mononuclear cells), saliva, urine, stool (i.e., faeces), tears, sweat, sebum, nipple aspirate, ductal lavage, tumour exudates, synovial fluid, cerebrospinal fluid, lymph, fine needle aspirate, amniotic fluid, any other bodily fluid, cell lysates, cellular secretion products, inflammation fluid, semen and vaginal secretions. The sample may be whole blood or a fractional component thereof such as plasma, serum, or a cell pellet. Preferably the sample is readily obtainable by minimally invasive methods. Samples may also include tissue samples and biopsies, tissue homogenates and the like.

In this respect, the specification provides for a method for establishing a healthy reference biomarker profile comprising the steps of:
(a) determining a quantity of at least two biomarkers selected from the group consisting of Histone proteins, soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP) in a sample obtained from a subject not having a condition related to sepsis or SIRS; and
(b) storing the quantity of the selected biomarkers in the healthy subject sample in the form of a reference biomarker profile.

In addition, the specification provides for a method for establishing a SIRS reference biomarker profile comprising the steps of:
(a) determining a quantity of at least two biomarkers selected from the group consisting of Histone proteins, soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP)in a sample obtained from a subject having a condition of SIRS; and
(b) storing the quantity of the selected biomarkers in the healthy subject sample in the form of a reference biomarker profile.

Alternatively, the specification provides for a method for establishing a sepsis reference biomarker profile comprising the steps of:
(a) determining a quantity of at least two biomarkers selected from the group consisting of Histone proteins, soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP)in a sample obtained from a subject having a condition related to sepsis; and
(b) storing the quantity of the selected biomarkers in the healthy subject sample in the form of a reference biomarker profile.

### Kits

The specification also provides kits for predicting, prognosing and/or diagnosing sepsis in a subject. The kits as taught herein comprise at least one biomarker as defined herein or molecules specifically binding thereto. Specific biomarkers that are useful in the methods and kits as taught herein are those selected from the group of Histone proteins, soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP) but a kit may include one or two or three or four or all of the biomarkers listed therein with or without other biomarkers in addition.

The biomarker or biomarkers in each kit may be part of an array, or the biomarker(s) may be packaged separately and/or individually. The kit may also comprise at least one standard to be used in generating the biomarker profiles as described herein. The kits as taught herein also may contain reagents that can be used to detectably label biomarkers contained in the biological samples from which the biomarker profiles are generated. For this purpose, the kit may comprise a set of antibodies or functional fragments thereof that specifically bind to one or more of the biomarkers set forth in Table 1 and/or any other biomarkers that are included in creating the profile.

The specification also provides a method and a kit for assessing the occurrence and stage or severity of sepsis in a subject, which can range from the very onset of sepsis, to septic shock and eventually the death of the subject, by measuring the quantity of one or of a combination of one or more of the biomarkers as defined herein selected from the group of histone proteins soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP) in combination with known biomarkers for sepsis or, in the sample from the subject and comparing the biomarker measurements to that of a sample obtained from a healthy or non-sepsis subject. The specification provides a means for a clinician to estimate the degree of sepsis at an initial assessment, and to monitor the change in status of the sepsis (worsening, improving, or remaining the same) based on the detected amount of the one or more biomarkers in the sample of the subject.

Typically, the clinician would establish a protocol of collecting and analyzing a quantity of fresh sample from the patient at selected intervals. Typically the sample is obtained intermittently during a prescribed period. The period of time between intermittent sampling may be dictated by the condition of the subject, and can range from a sample each 24 hours to a sample taken continuously, more typically from each 4 hours to each 30 minutes.

Using the methods and techniques described herein, both a qualitative level of one or more of the biomarkers present in the sample can be analyzed and estimated, and a quantitative level of one or more of the biomarkers present in the sample can be analyzed and measured. The clinician would select the qualitative method, the quantitative method, or both, depending upon the status of the patient. Typically, the quantity of sample to be collected is less than 10 milliliter, less than 1 milliliter, and more typically less than 10 µl. A typical sample can range from about 1 µl to about 1 ml. Typically the larger quantities of sample (about 10 ml) are used for quantitative assays. Typically, these small amounts of sample are easily and readily available or obtainable from clinical subjects who are either prone to developing sepsis, or have developed sepsis.

Once an indication of sepsis has been detected, and intervention and treatment of the disease or condition has commenced, the clinician can employ the method and kit as taught herein to monitor the progress of the treatment or intervention. Typically, one or more subsequent post-treatment samples will be taken and analyzed for the presence of one or more of the biomarkers as the treatment of the sepsis condition commences and continues. The treatment is continued until the presence of one or more of the biomarkers as defined herein in subsequent post-treatment samples is normalized when compared to a sample obtained from a healthy or non-sepsis subject. As the treatment and intervention ameliorate the condition, the expression of one or more of the biomarkers, and its presence in the sample, will be altered and normalized when compared to a sample of a healthy or non-sepsis subject. The degree of amelioration will be expressed by a correspondingly normalized level of one or more of the biomarkers, detected in a sample. As the condition nears complete recovery, the method can be used to detect the complete normalization of one or more of the biomarkers as defined herein, signaling the completion of the course of treatment.

The term "binding molecules" refers to all suitable binding molecules that are specifically binding or interacting with one of the biomarkers as defined herein and that can be used in the methods and kits as taught herein. Examples of suitable binding agents are antibodies, aptamers, specifically interacting small molecules, specifically interacting proteins, and other molecules that specifically bind to one of the biomarkers. Both monoclonal and polyclonal antibodies that bind one of the biomarkers as defined herein are useful in the methods and kits as taught herein. The monoclonal and polyclonal antibodies can be prepared by methods known in the art and are often commercially available.

Aptamers that bind specifically to the biomarkers as defined herein can be obtained using the so called SELEX or Systematic Evolution of Ligands by EXponential enrichment. In this system, multiple rounds of selection and amplification can be used to select for DNA or RNA molecules with high specificity for a target of choice, developed by Larry Gold and coworkers and described in US patent 6,329,145. Recently a more refined method of designing co-called photoaptamers with even higher specificity has been described in US patent 6,458,539 by the group of Larry Gold.

Methods of identifying binding agents such as interacting proteins and small molecules are also known in the art. Examples are two-hybrid analysis, immunoprecipitation methods and the like.

Typically, the step of detecting the complex of the capture antibody and one or more of the biomarkers comprises contacting the complex with a second antibody for detecting the biomarker.

The method for detecting the complex of one or more of the biomarkers and the primary antibody or binding molecule comprises the steps of: separating any unbound material of the sample from the capture antibody-biomarker complex; contacting the capture antibody-biomarker complex with a second antibody for detecting the biomarker, to allow formation of a complex between the biomarker and the second antibody; separating any unbound second antibody from the biomarker-second antibody complex; and detecting the second antibody of the biomarker-second antibody complex.

A kit for use in the method typically comprises one or more media having affixed thereto one or more capture antibodies or binding molecules, whereby the sample is contacted with the media to expose the capture antibody or binding molecule to the biomarker present in the sample. The kit includes an acquiring means that can comprise an implement, such as a spatula or a simple stick, having a surface comprising the media. The acquiring means can also comprise a container for accepting the sample, where the container has a sample-contacting surface that comprises the media. In another typical example, the assay for detecting the complex of one or more of the biomarkers and the antibody or binding molecule can comprise an ELISA, and can be used to quantitate the amount of one or more the biomarkers in a sample. In an alternative example, the acquiring means can comprise an implement comprising a cassette containing the media.

Early detection of one or more of the biomarkers as defined herein can provide an indication of the presence of the protein in a sample in a short period of time. Generally, a method and a kit as taught herein can detect the biomarker in a sample within four hours, more typically within two hours, and most typically within one hour, following the sepsis condition. Preferably, the biomarker can be detected within about 30 minutes following the sepsis condition.

A rapid one-step method of detecting one or more of the biomarkers as defined herein can reduce the time for detecting the sepsis condition. A typical method can comprise the steps of: obtaining a sample suspected of containing one or more of the biomarkers; mixing a portion of the sample with one or more detecting antibodies or binding molecules that each specifically bind to one of the biomarkers, so as to initiate the binding the detecting antibody or binding molecule to the biomarkers in the sample; contacting the mixture of sample and detecting antibody or binding molecule with an immobilized capture antibody or binding molecule which specifically binds to the biomarker, which capture antibody or binding molecule does not cross-react with the detecting antibody or binding molecule, so as to bind the detecting antibody or binding molecule to the biomarker, and the biomarker to the capture antibody or binding molecule, to form a detectable complex; removing unbound detecting antibody or binding molecule and any unbound sample from the complex; and detecting the detecting antibody or binding molecule of the complex. The detectable antibody or binding molecule can be labeled with a detectable marker, such as a radioactive label, a fluorescent label, an enzyme label, a biological dye, a magnetic bead, (strept)avidin, or biotin, as is well known in the art.

### Use of the methods and kits as taught herein in treatment and diagnosis, prediction and/or prognosis

### Diagnosis, prediction, and/or prognosis of sepsis and sepsis versus SIRS

The specification provides a method for the prediction, prognosis and/or diagnosis of sepsis or the differentiation between SIRS and sepsis in a subject comprising obtaining a candidate biomarker profile from a biological sample taken from said subject wherein said candidate biomarker profile is based on the measurement of the quantity of Histone proteins in said sample, and comparing said candidate biomarker profile with a reference biomarker profile obtained form a healthy subject or a patient having SIRS.

Also provided by the specification is a method for the prediction, prognosis and/or diagnosis of sepsis or for the differentiation between SIRS and sepsis in a subject comprising: obtaining a candidate biomarker profile from a biological sample taken from said subject wherein said candidate biomarker profile is based on at least one or two biomarkers selected from the group consisting of Histone proteins, soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP) and comparing said candidate profile with a reference biomarker profile obtained form a healthy subject or a patient having SIRS.

The specification further provides for a method for the prediction, prognosis and/or diagnosis of sepsis or for the differentiation between SIRS and sepsis in a subject comprising measuring the level of at least one or two biomarkers selected from the group consisting of Histone proteins, soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP) in a biological sample from said subject; using said measurements obtained in step a) to create a profile for said biomarkers; and comparing said profile with a reference biomarker profile obtained form a healthy subject or a patient having SIRS.

The specification further provides for a method for the prediction, prognosis and/or diagnosis of sepsis or for the differentiation between SIRS and sepsis in a subject comprising determining a quantity of at least one or two biomarkers selected from the group consisting of Histone proteins, soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP) in a sample obtained from a subject; and comparing the quantity of the selected biomarkers in the test subject sample with a range of normal values of the selected biomarkers in control subjects; whereby an increase or decrease in the quantity of the selected biomarker in the sample to a level higher or lower than the range of normal values of the selected biomarkers is indicative of sepsis.

The specification further provides for a method for the prediction, prognosis and/or diagnosis of sepsis or for the differentiation between SIRS and sepsis in a subject comprising determining a quantity of at least one or two biomarkers selected from the group consisting of Histone proteins, soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP) and comparing the quantity of the selected biomarkers in the test subject sample with a range of values of the selected biomarkers obtained from subjects with sepsis; whereby a comparable quantity of the selected biomarkers in said sample to the range of values of the selected biomarkers in subjects with sepsis is indicative of sepsis.

Alternatively, the specification provides for a method for the prediction, prognosis and/or diagnosis of sepsis or for the differentiation between SIRS and sepsis in a subject comprising obtaining a candidate antibody profile from a biological sample taken from said individual wherein said candidate antibody profile is based on an antibody to at least one or two biomarkers selected from the group consisting of Histone proteins, soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP) and comparing said candidate antibody profile with a reference antibody profile.

The specification further provides for a method for determining whether an individual is responsive to treatment for sepsis with a substance, comprising the steps of obtaining a candidate biomarker profile from a biological sample taken from said individual wherein said candidate biomarker profile is based on at least one or two biomarkers selected from the group consisting of Histone proteins, soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP) and comparing said candidate profile with a reference biomarker profile.

The selected biomarker as described herein comprises one or more members of the Histone protein family. Alternatively, this marker or these markers are combined with any one or more of the other biomarkers provided herein selected from the group of: soluble TNF-receptor 2 (sTNFR2), Pentraxin-3 (PTX-3), Macrophage Colony-Stimulating Factor (MCSF), pro-Hepcidin (pro-HEPC), pro-Brain Natriuretic Protein (pro-BNP), Procalcitonin (PCT) and c-Reactive Protein (CRP)

Preferably, said Histone proteins are selected from the group of Histone H1.4, Histone H2A (different isoforms), Histone H2B (different isoforms), Histone H3 (different isoforms), Histone H4 (different isoforms).

For example, the selected biomarkers are pro-Hepcidin (pro-HEPC) and Pentraxin-3 (PTX-3).

Preferred samples to be analysed in the method as described herein are blood or urine, more preferable the sample is serum or plasma.

The method as described herein uses immunoassay technology selected from the group of direct ELISA, indirect ELISA, sandwich ELISA, competitive ELISA, multiplex ELISA, radioimmunoassay, or ELISPOT technologies to establish the biomarker profile. Alternatively, the biomarker profile is established using mass spectrometry analysis methods of the proteins present in said sample.

The methods indicated herein are particularly useful to distinguish between SIRS and sepsis.

### Treatment

Once a condition of sepsis has been diagnosed, the identification of the biomarkers as defined herein could be of use in the treatment or amelioration of the sepsis condition of the subject.

It is possible to increase the expression level or abundance of a protein in a subject by administrating such a purified, synthetically or recombinantly produced biomarker as defined herein to a subject having a reduced level of said biomarker in comparison to a healthy subject. Administering agents that increase the expression or activity of said biomarker may also be beneficial to the patient. The presence of the sTNFR2 biomarker in blood obtained from a subject having sepsis for example is drastically reduced when compared to the samples of a subject having SIRS or a healthy subject.

Another possibility can be the reduction of the level or abundance of a certain biomarker as defined herein in case said biomarker has an increased occurrence in the blood of patients having sepsis when compared to the samples of a subject having SIRS or a healthy subject. Examples of these biomarkers are Histone proteins, pro-BNP, PTX-3, pro-HEPC and M-CFS. Administering agents that reduce the expression or activity of said proteins may be beneficial to the subject.

### Examples

The following experimental details describe the complete exposition of one example of the invention as described above and are not to be deemed limiting in any way.

### Example 1: Identification of new biomarkers for sepsis

In order to find new biomarkers to diagnosis sepsis or SIRS, we determined the protein profile of blood samples obtained from healthy human subjects and samples from subjects having sepsis or SIRS without sepsis. The analysis was done using mass spectrometric detection of protein levels using our previously published COFRADIC^{™} technology platform.

In a reference design study each sample is measured against a reference sample, typically a pool of all patient samples. For each feature present in a certain sample a ratio is obtained which represents the fold difference of the feature intensity in the reference versus feature intensity in the sample. Combining all feature data from all samples into an expression matrix allows comparing features intensities between samples and between groups of samples.

For the SIRS/SEPSIS a 10-10 reference design study was set up and hence the reference pool was a pool of all 20 samples. An intermediate matrix analysis was performed after 3 samples of each group were run through the platform.

All serum samples were delipidated followed by depletion for the most abundant proteins using a Beckman Ig-Y12 column. Depletion efficiency was checked using ELISAs and Western Blot analysis. The reference pool was prepared at this stage and this reference was considered as a normal sample for the rest of process. Samples were prepared for MASStermind analysis according the standard N-ter COFRADIC procedures. Reference pool and samples were differentially labeled by trypsin mediated incorporation of ¹⁸O/¹⁶O, where the different samples carried the heavy oxygen label and the reference the ¹⁶O. Just before COFRADIC sorting each sample was mixed with the reference at equal protein masses. After sorting, NanoLC separations MS spectra were obtained. MS data were deisotoped, clustered and features were constructed using in house developed software called euCatLabel. The output of this data processing is an expression matrix containing all features from all analyzed samples. Each feature is represented by a unique combination of m/z, COFRADIC sorting pool and NanoLC retention time and features can be present in a number of samples ranging from 1 to all. If a feature is present in a sample it will carry a ratio, which represents intensity of the feature in the reference sample (ie the ¹⁶O peak) over the intensity in the respective sample (the ¹⁸O peak). Recurrent quantifiable features are features with a reliable ratio reading in at least one sample. For the intermittent matrix analysis of 3 samples each, all features were considered that were either present in 5/6 samples or were present in all 3 samples of the SIRS patient group and in none of the samples of the SEPSIS patient group.

Upon comparison of the protein profiles, we were able to identify six biomarkers that were present in differing amounts in the blood of patients with and without sepsis and looked promising as differentiating biomarkers.

The six identified biomarkers are:
X1: soluble TNF-receptor 2 (sTNFR2),,
X2: Pentraxin 3 (PTX-3),
X3: Macrophage Colony Stimulating Factor (M-CSF),,
X4: Pro-HEPC (Hepcidin) and
X5: Brain Natriuretic Protein (pro-BNP), and
X6: Several Histones or Histone fragments (cf table 1b)

### Example 2: ELISA measurements of the newly identified biomarkers as defined herein in samples from patients suffering from SIRS or several stages of sepsis.

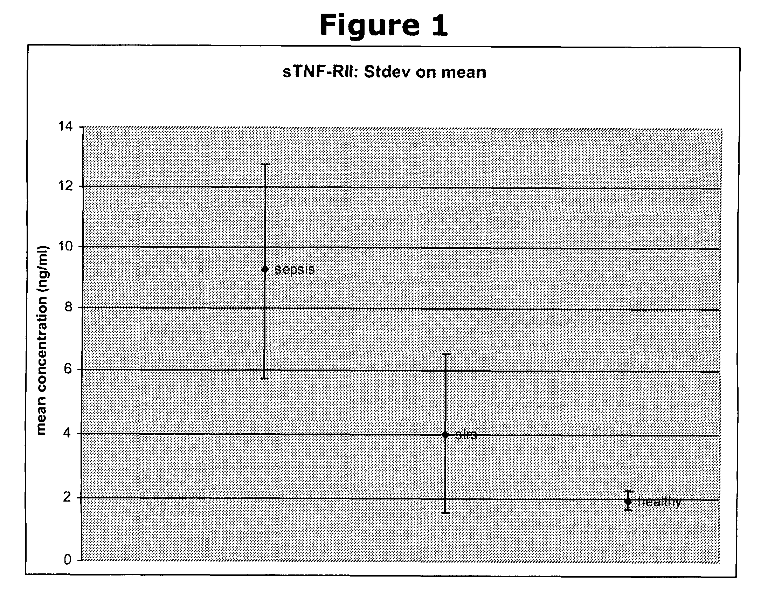

In a next experiment, the biomarkers obtained from the Cofradic analysis were analysed for their differential expression in blood samples from patients suffering from SIRS or several stages of sepsis, using ELISA with specific antibodies directed to each of the biomarkers. As can be seen in Table 2 of the present application, we compared the quantity of these five protein biomarkers in the different blood samples with two known SIRS / sepsis biomarkers procalcitonin (PCT) and c-reactive protein (CRP). From the table it becomes clear that markers X1-X5 show a clear difference in protein quantity between samples from healthy subjects and the samples form subjects suffering from SIRS or sepsis. The results are shown in Figures 1-5 for X1-X5 respectively.

For the detection of human Pentraxin-3, we used a monoclonal mouse anti-human Pentraxin 3 antibody (R&D systems) as a capture antibody attached to a solid phase and a biotinylated goat anti-human Pentraxin 3 antibody as a detection antibody. Sepsis and SIRS samples were diluted as follows: 22µl sample + 198µl assay buffer (load 100µl/well). Healthy samples were diluted in a ½ ratio.

The Human Hepcidin Prohormone was detected using a polyclonal anti human Pro-Hepcidin antibody (DRG) as a capturing antibody and a Pro-Hepcidin fragment conjugated to biotin as a conjugate in a competitive ELISA set-up. Sepsis and SIRS samples are diluted as follows: 60µl sample + 60µl assay buffer (load 50 µl/well). Healthy samples were diluted in a ½ ratio.

For the detection of human Pro-BNP, a polyclonal anti-BNP fragment (8-29) antibody (Biomedica) was used as a capturing antibody and a synthetic BNP fragment coupled to HRP was used as a conjugate in a competitive ELISA set-up. Sepsis and SIRS samples are diluted as follows: 150µl sample + 300µl assay buffer (load 200µl/well). Healthy samples were diluted in a ½ ratio.

M-CSF was detected by using a mouse monoclonal antibody against human M-CSF(R&D systems) as a capturing antibody and a polyclonal antibody against human M-CSF conjugated to HRP as a detection antibody. Sepsis and SIRS samples are diluted as follows: 44µl sample +176µl assay buffer (load 100µl/well). Healthy samples were diluted in a ½ ratio.

Finally, sTNF-RII was detected using a mouse monoclonal antibody against human sTNF-RII (R&D systems) as a capture antibody and a polyclonal antibody against human sTNF-RII conjugated to HRP as a detection antibody. Sepsis and SIRS samples are diluted as follows: 14µl sample + 406µl assay buffer (load 200µl/well). Healthy samples were diluted in a ½ ratio.

| **Table1: Concentration determination in 20 samples using ELISA assays** | | | | | | | |
|---|---|---|---|---|---|---|---|
| | ***Known markers*** | | ***Newly identified biomarkers*** | | | | |
| **Marker** | **PCT** (ng/ml) | **CRP** (µg/ml) | **Pro-BNP** (ng/ml) | **PTX-3** (ng/ml) | **Pro-HEPC** (ng/ml) | **sTNFR2** (ng/ml) | **M-CSF** (ng/ml) |
| **Sepsis (n=11)** | 53 | 287 | 9,3 | 47,7 | 2,8 | 102 | 9,7 |
| **SD** | | | 3,5 | 28,5 | 1,9 | 40 | 6,3 |
| **SIRS (n=9)** | 6 | 236 | 4,0 | 8,8 | 1,4 | 157 | 3,3 |
| **SD** | | | 2,5 | 4,5 | 1,1 | 49 | 1,4 |
| **Healthy (n=4)** | | | 1,9 | 0,9 | 0,3 | 339 | 2,8 |
| **SD** | | | 0,3 | 0,3 | 0,1 | 27 | 0,5 |
| n = number op subjects tested / SD = Standard Deviation | | | | | | | |

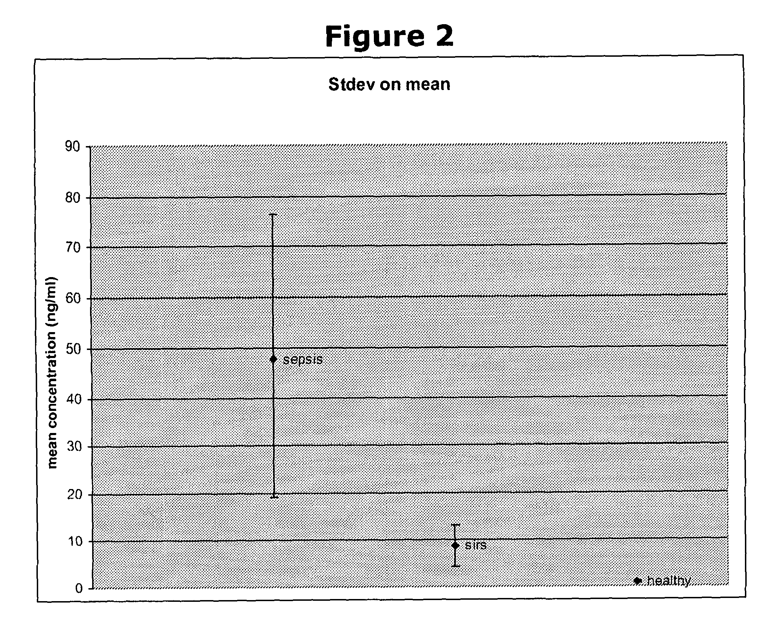

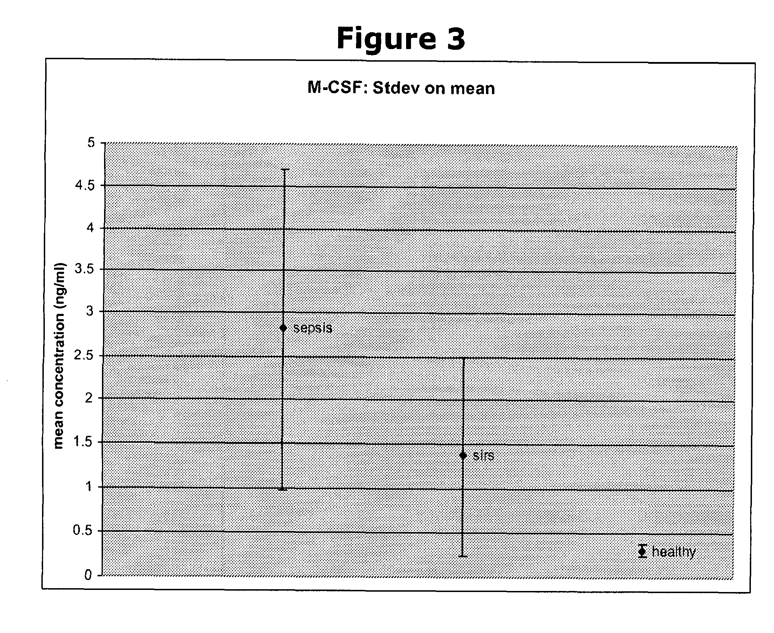

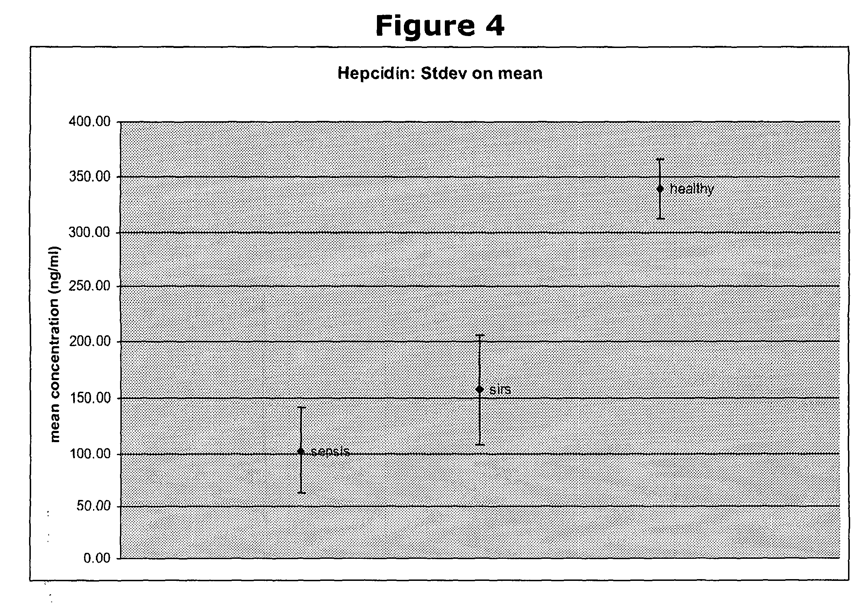

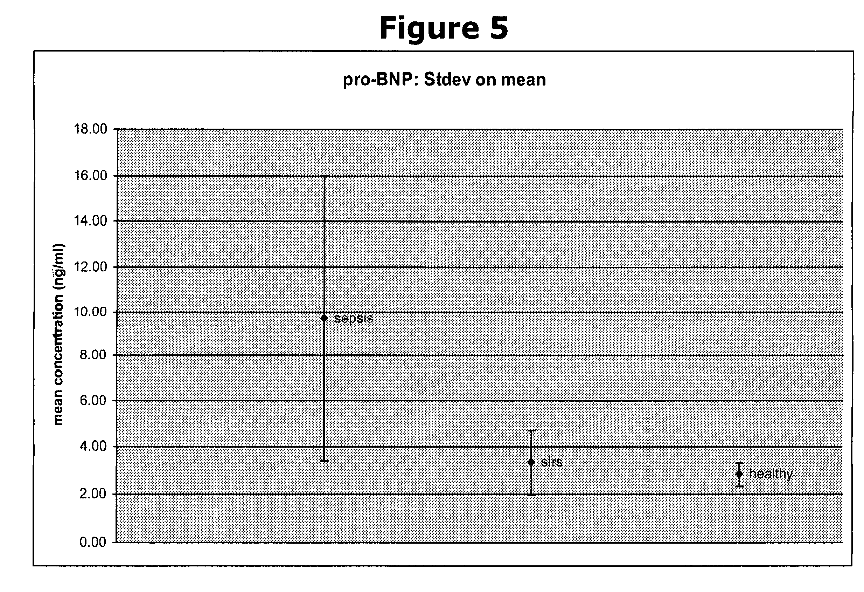

As can be derived from Figures 1-5, the biomarkers as defined herein are all valuable for distinguishing SIRS conditions from sepsis conditions. Of the five identified markers as defined herein, pro-BNP (Figure 5), PTX3 (Figure 2) and pro-HEPC (Figure 4) appear to be the most valuable in differentiating between sepsis and SIRS, while the results for M-CSF and sTNFR2 are less convincing due to high variations in the measurements between different patients (cf. Figures 3 and 1). Although the number of patients tested is still low, we believe the markers identified herein show very promising results and will be very useful in the development of new diagnostic tools to distinguish between sepsis and SIRS.

### Example 3: Testing the predictive characteristics of different combinations of the identified biomarkers.

In order to test the predictive power of the identified markers, we employed a classification framework as opposed to a summarizing statstical approach. A classifier is induced from the data that implements a model that can then be used to predict the class (sepsis/sirs) of a new patient. The generalization error (an estimate of how the classifier will perform on new patients) is computed using Leave-One-Out error. The data set of the ELISA measurements is given in Table 3. Note that we included ELISA measurements of both PCT and CRP, two known and widely used biomarkers for sepsis.

| **Table 3: Data set of the ELISA measurements (conc. In ng/ml)** | | | | | | | | |
|---|---|---|---|---|---|---|---|---|
| | **Marker** | **PCT** | **CRP** | **sTNFR2** | **PTX-3** | **M-CSF** | **Pro-HEPC** | **pro-BNP** |
| **Patient** | **Class** | | | **X4** | **X2** | **X5** | **X3** | **X1** |
| HGE67 | SEPSIS | 128.5 | 369 | 12.25 | 54.015 | 5.91 | 52.62 | 9.84 |
| UGE22 | SEPSIS | 226.1 | 224 | 9.59 | 72.085 | 2.727 | 138.19 | 18.21 |
| HGE74 | SEPSIS | 5.2 | 335 | 6.82 | 17.937 | 2.022 | 73.9 | 3.86 |
| UGE41 | SEPSIS | 4.5 | 347 | 7.89 | 15.945 | 3.62 | 105.78 | 5.71 |
| UGE10 | SEPSIS | 27.37 | 197 | 11.1 | 26.519 | 1.17 | 133.27 | 21.46 |
| UGE14 | SEPSIS | 51.00 | 297 | 9.96 | 53.807 | 5.69 | 155.26 | 9.13 |
| UGE50 | SEPSIS | 23.43 | 288 | 15.74 | 40.763 | * | 62.32 | 0.00 |
| HGE73 | SEPSIS | 46.70 | 225 | 4.3 | 16.94 | 1.19 | 82.69 | 4.68 |
| UGE45 | SEPSIS | 9.33 | 247 | 5.22 | 49.435 | 1.11 | 114.36 | 4.71 |
| HGE63 | SEPSIS | 46.76 | 257 | 12.66 | 68.764 | * | 153.57 | 0.00 |
| HGE81 | SEPSIS | 11.73 | 373 | 6.24 | 108.419 | 2.07 | 52.01 | 8.79 |
| UGE12 | SIRS | 2.46 | 174 | 2.54 | 7.012 | 0.91 | 114.81 | 2.71 |
| UGE17 | SIRS | 1.41 | 177 | 1.72 | 6.837 | * | 173.35 | 0.00 |
| UGE20 | SIRS | 9.62 | 280 | 2.16 | 3.007 | 0.644 | 220.93 | 1.78 |
| UGE32 | SIRS | 24.86 | 286 | 5.34 | 17.158 | 0.844 | 149.96 | 2.47 |
| UGE40 | SIRS | 1.10 | 155 | 9.44 | 13.575 | 2.006 | 85.1 | 5.42 |
| UGE42 | SIRS | 1.39 | 328 | 3.44 | 7.476 | 0.621 | 186.43 | 4.20 |
| UGE46 | SIRS | 2.99 | 412 | 6.15 | 12.197 | 3.91 | 203.89 | 2.93 |
| UGE53 | SIRS | 7.30 | 108 | 2.83 | 5.366 | 0.64 | 182.65 | 4.84 |
| UGE60 | SIRS | 0.21 | 208 | 2.63 | 6.953 | 1.35 | 95.13 | 2.07 |
| * Missing MCF data is imputed by column mean. | | | | | | | | |

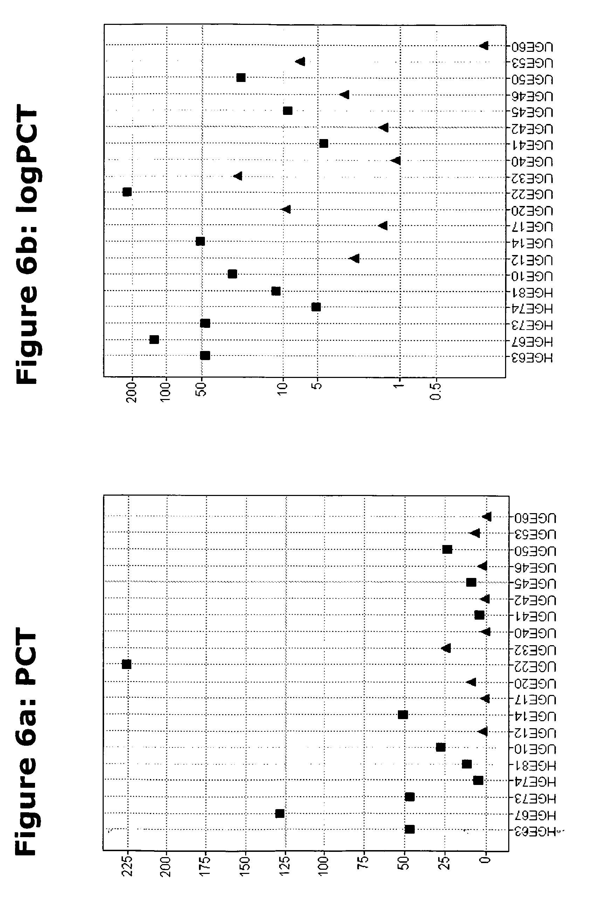

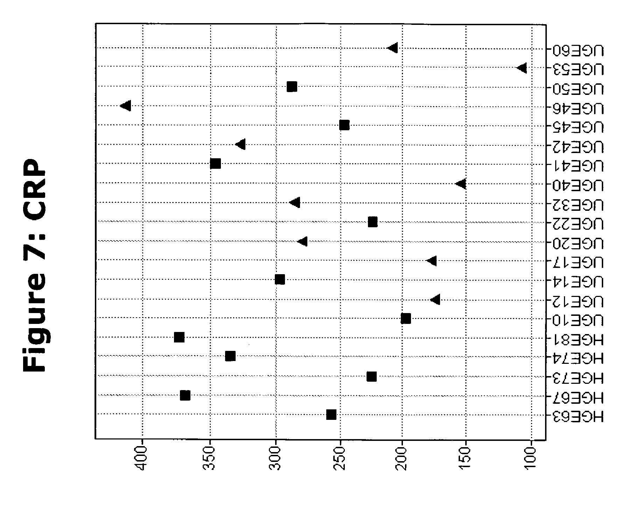

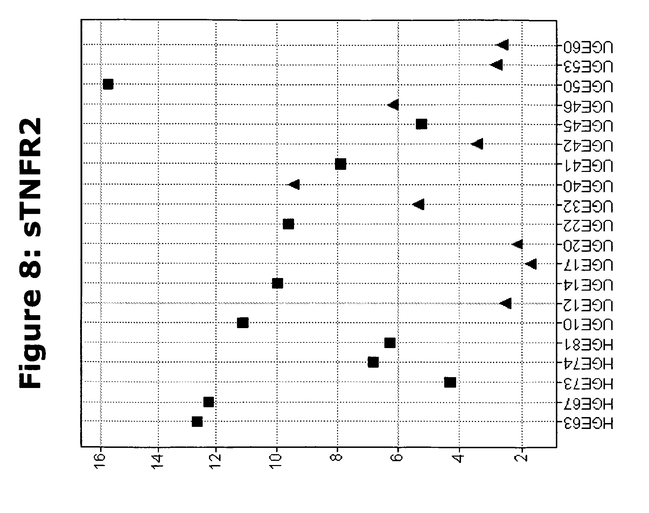

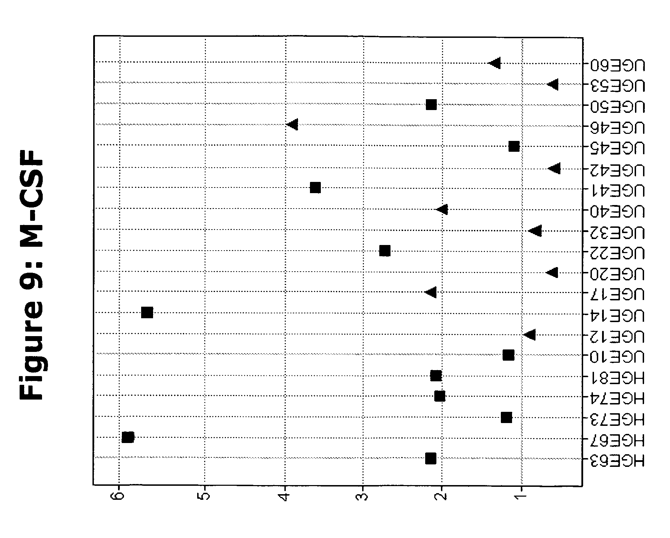

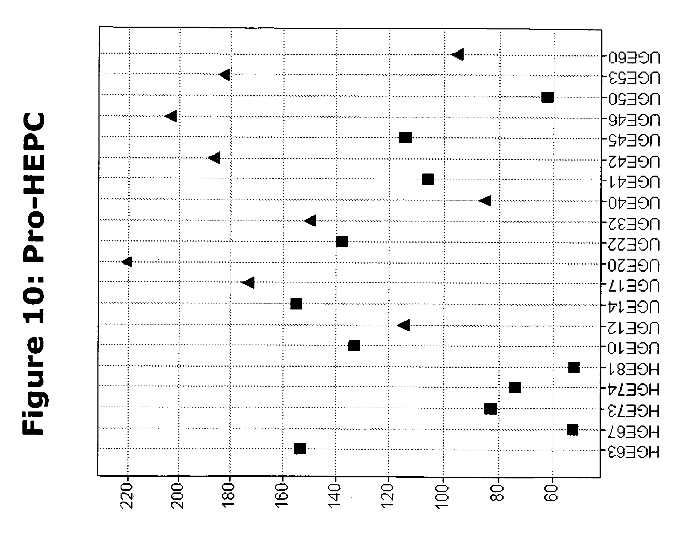

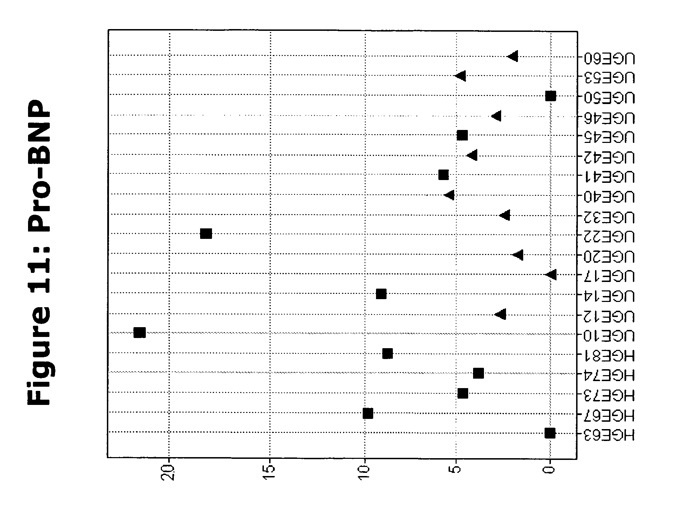

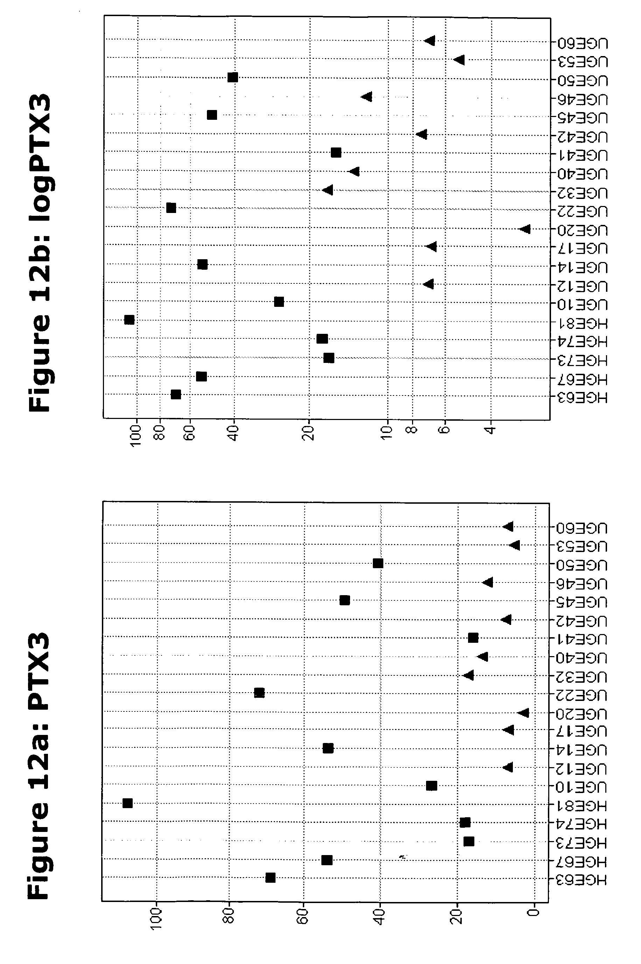

We predicted the ability of the different biomarkers, either alone or in combination to differentiate between blood samples obtained from patients having SIRS or sepsis. The results are given in Figures 6-12. The plots show ELISA measurements for PCT, CRP, proHepcidin, MCSF, proBNP, TNRF2 and PTX3 (in that order). Rectangles represents sepsis, triangles represents SIRS. The black line shows the optimal threshold computed from all patients using entropy.

To predict the differentiating power of the markers, we used a simple linear classifier induced from the data using one protein only. Decision trees represent a supervised approach to classification. A decision tree is a simple structure where non-terminal nodes represent tests on one or more attributes and terminal nodes reflect decision outcomes. J.R. Quinlan has popularized the decision tree approach with his research spanning more than 15 years. The latest public domain implementation of Quinlan's model is C4.5. The Weka classifier package has its own version of C4.5 known as J48. Program 1R is ordinary in most respects. It ranks attributes according to error rate (on the training set), as opposed to the entropy-based measures used in C4. It treats all numerically-valued attributes as continuous and uses a straightforward method to divide the range of values into several disjoint intervals. It handles missing values by treating "missing" as a legitimate value.

As explained above we can compute the generalization error of such classifier using LOO:

| *protein* | *J48* | *OneR* |
|---|---|---|
| PCT | 20% | **15%** |
| CRP | **35%** | 45% |
| sTNRF2 | 30% | **20%** (corrected) |
| MCSF | **25%** | 35% |
| pro-HEPC | **25%** | 40% |
| pro-BNP | **30%** | **30%** |
| PTX3 | 20% | **5%** |

So, as indvidual protein PTX3 seems to provide best discrimination using the OneR classifier induction method, followed by PCT. We also observe that there is no one best induction method. The difference between both methods is mainly in the way the optimal threshold value is computed.

Next, we looked at combinations of proteins. For this we employ a Linear Support Vector Machine (LSVM) which implements a large margin classifier induction method. The LSVM induces a classifier from the data that is very similar to the black line classifer shown above. But the linear threshold is computed in a 7-dimensional space (the space of the 7 ELISA measurments) instead of a 1-dimensional space. As such, the classifier is not a simple line but a hyperplane in the 7-dimensional space. The LSVM classifier achieves a 15% LOO error when using all 7 elisa measurements (this differs from the 10% reported before due to swaps in the MCSF data).

Next, a feature subset evaluation method was used to rank the proteins according combined discrimination performance. As we have very few data, the LSVM cost parameter C is set to a default value 1. The result:

### Run information:

**Evaluator:** weka.attributeSelection.ClassifierSubsetEval -B weka.classifiers.functions.SMO -T -H "Click to set hold out or test instances" -C 1.0 -E 1.0 -G 0.01 -A 250007 -L 0.0010 -P 1.0E-12 -N 0 -V -1 -W 1
**Search:** weka.attributeSelection.ExhaustiveSearch
**Relation:** ELISA-weka.filters.unsupervised.attribute.Remove-R1-weka.filters.unsupervised.attribute.Normalize
   - **Instances:**: 20
   - **Attributes:**: 8
   PCT
   CRP
   sTNFR2
   PTX3
   MCSF
   proHEPC
   pro-BNP
   class
**Evaluation mode:** 20-fold cross-validation

### Attribute selection 20 fold cross-validation (stratified), seed: 1 number of folds (%) attribute

| | |
|---|---|
| 5(25 %) | 1 PCT |
| 9(45 %) | 2 CRP |
| 9(45 %) | 3 sTNFR2 |
| **14(70 %)** | 4 PTX3 |
| 3(15 %) | 5 MCSF |
| **17(85 %)** | 6 proHEPC |
| 7(35 %) | 7 pro-BNP |

Suprisingly, pro-Hepcidin is most relevant. This indicates that it is complementary to the other proteins. PTX3 also shows nice combined discrimination power.

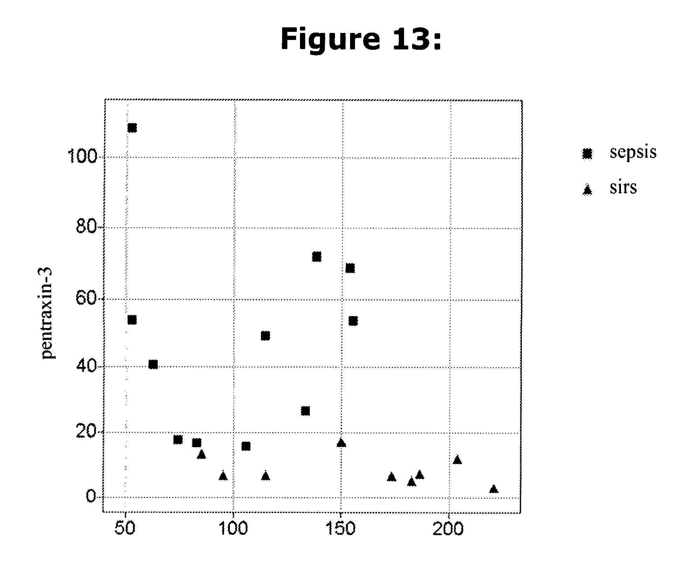

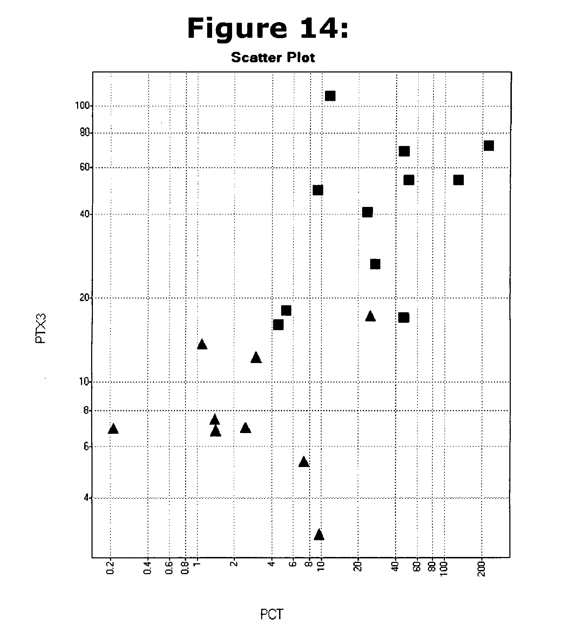

Using PTX3 and pro-HEPC in a LSVM classifier achieves 10% LOO error. Adding any of the individual protein increases LOO error. The plot in Figure 13 shows ELISA measurements for both proteins (PTX3 = y-axis; Pro-HEPC = x-axis), showing some complementary behaviour between both proteins. Using PCT, pro-HEPC and sTNRFR2 in an LSVM classifier also achieves 10% LOO error. The plot in Figure 14 shows some correlation between PCT and PXT3 (PTX3 = y-axis). In conclusion, we see that PTX3 biomarker shows the best individual generalization error, better than the known PCT biomarker. The combination of PTX3 and pro-HEPC perfoms best, the subsampling procedure shows acceptable LOO error variance for the PTX3 + pro-HEPC classifier. A combination of the known marker PCT with the pro-HEPC and sTNFR2 biomarkers performs good.

### Example 4: Testing the predictive power of the histone components

Also histone proteins were selected for further validation studies however there are no ELISAs available, hence MS based verifcation assays (MRM) have been set-up. MRM (Multiple Reaction Monitoring) assay approach has previously been applied to the measurement of specific peptides in complex mixtures such as tryptic digests of plasma. Such an assay requires only knowledge of the masses of the selected peptide and its fragment ions, and an ability to make the stable isotope-labeled version.

Since the sensitivity of these assays is limited by mass spectrometer dynamic range and by the capacity and resolution of the assisting chromatography separation(s), we could also use hybrid methods coupling MRM assays with enrichment of proteins by immunodepletion and size exclusion chromatography or enrichment of peptides by antibody capture. In essence the latter approach uses the mass spectrometer as a "second antibody" that has absolute structural specificity. It has been shown to extend the sensitivity of a peptide assay by at least two orders of magnitude and with further development appears capable of extending the MRM method to cover the full known dynamic range of plasma (i.e., to the pg/ml level).

Several publications and patent documents are referenced in this application in order to more fully describe the state of the art to which this invention pertains.

## Claims

1. The use of Histone proteins as biomarkers for diagnosis, prognosis and/or prediction of sepsis versus systemic inflammatory response syndromes (SIRS) in a blood sample of a human subject.

2. A method for distinguishing between a condition of sepsis and systemic inflammatory response syndromes (SIRS) in a human subject comprising:
(a) measuring the level of Histone proteins in a blood sample taken from said subject; and
(b) comparing said level of Histone proteins with a reference level of said Histone proteins obtained from a patient having SIRS, wherein the Histone proteins are preferably selected from the group consisting of Histone H1.4, Histone H2A, Histone H2B, Histone H3, Histone H4, and isoforms thereof, wherein the increased detection of Histone proteins in the sample as compared to said reference level indicates the patient is at risk of suffering from sepsis or severe sepsis rather than SIRS.

3. A method to monitor the progress of the treatment or intervention of a human subject in which sepsis has been detected, or for determining whether a human subject in which sepsis has been detected is responsive to treatment for sepsis, comprising measuring the quantity of one or more Histone proteins in blood samples of the subject taken at different time points during the treatment, wherein preferably said Histone proteins are selected from the group consisting of: Histone H1.4, Histone H2A, Histone H2B, Histone H3, Histone H4, and isoforms thereof.

4. The method according to claim 3, wherein a normalization of said one or more Histone protein levels in said sample indicates recovery from sepsis,

5. The method according to claim 3, wherein an altered Histone protein profile in the sample taken during treatment is indicative of the responsiveness of the subject to treatment with the substance.

6. The use of a kit comprising:
a) one or more binding molecules to Histone proteins, selected from the group consisting of: Histone H1.4, Histone H2A, Histone H2B, Histone H3, Histone H4, and isoforms thereof; and
(b) a reference value of the quantity of the selected biomarkers in a patient having SIRS for comparison of the results, for the prediction, prognosis and/or diagnosis of sepsis versus SIRS, wherein the binding molecule is preferably selected from the group consisting of monoclonal antibodies, polyclonal antibodies, aptamers, photoaptamers, specific interacting proteins, and specific interacting small molecules.

7. The method according to claim 2, comprising:
(a) obtaining a candidate biomarker profile from a blood sample taken from said subject wherein said candidate biomarker profile is based on one or more Histone proteins, selected from the group consisting of Histone H1.4, Histone H2A, Histone H2B, Histone H3, Histone H4, and isoforms thereof, and (b) comparing said candidate profile with a reference biomarker profile.

8. The method according to claim 7, wherein the reference biomarker profile comprises a range of normal values of selected biomarkers in control subjects having SIRS, whereby an increase in the quantity of the selected biomarkers in the sample to a level higher than the range of normal values of selected biomarkers is indicative of sepsis, or wherein the reference biomarker profile comprises a range of values of selected biomarkers obtained from subjects with sepsis, whereby a comparable quantity of the selected biomarkers in said sample to the range of values of the selected biomarkers in subjects with sepsis is indicative of sepsis.

9. The method according to any one of the previous claims wherein the sample is serum, or plasma.

10. The method according to any one of the previous claims, wherein the histone protein level or biomarker profile is established using immunoassay technology selected from the group of direct ELISA, indirect ELISA, sandwich ELISA, competitive ELISA, multiplex ELISA, radioimmunoassay, or ELISPOT technologies, or using mass spectrometry (MS) analysis methods of the proteins present in said sample, preferably Multiple Reaction Monitoring (MRM).

## Patentansprüche

1. Verwendung von Histonproteinen als Biomarker zur Diagnose, Prognose und/oder Vorhersage von Sepsis gegenüber systemischen Entzündungsreaktionssyndromen (SIRS) in einer Blutprobe eines menschlichen Individuums.

2. Verfahren zur Unterscheidung zwischen einem Zustand der Sepsis und systemischen Entzündungsreaktionssyndromen (SIRS) in einem menschlichen Individuum, umfassend:
(a) Messen des Spiegels der Histonproteine in einer aus dem Individuum entnommenen Blutprobe; und
(b) Vergleichen des Spiegels der Histonproteine mit einem Referenzspiegel der Histonproteine, die aus einem Patient mit SIRS erhalten wurden, wobei die Histonproteine vorzugsweise ausgewählt sind aus der Gruppe, bestehend aus Histon H1.4, Histon H2A, Histon H2B, Histon H3, Histon H4, und Isoformen davon, wobei der erhöhte Nachweis von Histonproteinen in der Probe im Vergleich zu dem Referenzspiegel zeigt, dass der Patient gefährdet ist, an Sepsis oder schwerer Sepsis, statt an SIRS zu erkranken.

3. Verfahren zum Überwachen des Fortschritts der Behandlung oder Intervention eines menschlichen Individuums, bei dem Sepsis nachgewiesen wurde, oder zum Bestimmen, ob ein menschliches Individuum, bei dem Sepsis nachgewiesen wurde, auf die Sepsisbehandlung anspricht, umfassend das Messen der Menge von einem oder mehreren Histonproteinen in Blutproben des Individuums, die zu verschiedenen Zeitpunkten während der Behandlung entnommen wurden, wobei die Histonproteine vorzugsweise ausgewählt sind aus der Gruppe, bestehend aus: Histon H1.4, Histon H2A, Histon H2B, Histon H3, Histon H4, und Isoformen davon.

4. Verfahren nach Anspruch 3, wobei eine Normalisierung des einen oder mehreren Histonproteinspiegels in der Probe eine Erholung von der Sepsis anzeigt.

5. Verfahren nach Anspruch 3, wobei ein verändertes Histonproteinprofil in der Probe, die während der Behandlung entnommen wurde, die Ansprechbarkeit des Individuums auf die Behandlung mit der Substanz anzeigt.

6. Verwendung eines Kits, umfassend:
a) ein oder mehrere bindende Moleküle an Histonproteine, ausgewählt aus der Gruppe, bestehend aus Histon H1.4, Histon H2A, Histon H2B, Histon H3, Histon H4, und Isoformen davon; und
b) einen Referenzwert der Menge der selektierten Biomarker in einem Patient mit SIRS zum Vergleich der Ergebnisse, zur Vorhersage, Prognose und/oder Diagnose von Sepsis gegenüber SIRS, wobei das bindende Molekül vorzugsweise ausgewählt ist aus der Gruppe, bestehend aus monoklonalen Antikörpern, polyklonalen Antikörpern, Aptameren, Photoaptameren, spezifischen wechselwirkenden Proteinen, und spezifischen wechselwirkenden Kleinmolekülen.

7. Verfahren nach Anspruch 2, umfassend:
(a) Gewinnen eines Kandidaten-Biomarkerprofils aus einer Blutprobe, die aus dem Individuum entnommen wurde, wobei das Kandidaten-Biomarkerprofil auf einem oder mehreren Histonproteinen beruht, ausgewählt aus der Gruppe, bestehend aus Histon H1.4, Histon H2A, Histon H2B, Histon H3, Histon H4, und Isoformen davon, und (b) Vergleichen des Kandidatenprofils mit einem Referenz-Biomarkerprofil.

8. Verfahren nach Anspruch 7, wobei das Referenz-Biomarkerprofil einen Bereich von Normalwerten selektierter Biomarker in Kontrollindividuen mit SIRS umfasst, wobei ein Anstieg der Menge der selektierten Biomarker in der Probe auf einen höheren Wert als den Bereich der Normalwerte der selektierten Biomarker Sepsis anzeigt, oder wobei das Referenz-Biomarkerprofil einen Wertebereich selektierter Biomarker umfasst, die aus Individuen mit Sepsis erhalten wurden, wobei eine vergleichbare Menge der selektierten Biomarker in der Probe zum Wertebereich der selektierten Biomarker in Individuen mit Sepsis eine Sepsis anzeigt.

9. Verfahren nach einem der vorhergehenden Ansprüche, wobei es sich bei der Probe um Serum oder Plasma handelt.

10. Verfahren nach einem der vorhergehenden Ansprüche, wobei der Histonproteinspiegel oder das Biomarkerprofil mittels Immunassay-Technologie erbracht wird, ausgewählt aus der Gruppe mit direkten ELISA-, indirekten ELISA-, Sandwich-ELISA-, kompetitiven ELISA-, Multiplex-ELISA-, Radioimmunassay-, oder ELISPOT-Technologien oder unter Verwendung von Massenspektrometrie- (MS) -Analyse-Verfahren der in der Probe vorhandenen Proteine, vorzugsweise Multiple Reaction Monitoring (MRM).

## Revendications

1. Utilisation de protéines d'histones en tant que biomarqueurs pour le diagnostic, le pronostic et/ou la prédiction d'un état septique par rapport à des syndromes de réponse inflammatoire systémique (SRIS) dans un échantillon de sang d'un sujet humain.

2. Procédé pour distinguer un état septique et des syndromes de réponse inflammatoire systémique (SRIS) chez un sujet humain comprenant :
(a) la mesure du taux de protéines d'histones dans un échantillon de sang prélevé à partir dudit sujet ; et
(b) la comparaison dudit taux de protéines d'histones à un taux de référence desdites protéines d'histones obtenu à partir d'un patient atteint de SRIS, dans lequel les protéines d'histones sont de préférence choisies dans le groupe constitué de l'histone H1.4, l'histone H2A, l'histone H2B, l'histone H3, l'histone H4, et des isoformes de celles-ci, la détection accrue de protéines d'histones dans l'échantillon par rapport audit taux de référence indiquant que le patient est à risque de souffrir d'un état septique ou d'un état septique grave plutôt que SRIS.

3. Procédé pour surveiller le progrès du traitement ou l'intervention d'un sujet humain chez lequel un état septique a été détecté, ou pour déterminer si un sujet humain chez lequel un état septique a été détecté répond au traitement pour l'état septique, comprenant la mesure de la quantité d'une ou plusieurs protéines d'histones dans des échantillons de sang du sujet prélevés à différents temps pendant le traitement, dans lequel, de préférence, lesdites protéines d'histones sont choisies dans le groupe constitué de : l'histone H1.4, l'histone H2A, l'histone H2B, l'histone H3, l'histone H4, et des isoformes de celles-ci.

4. Procédé selon la revendication 3, dans lequel une normalisation desdits un ou plusieurs taux de protéine d'histone dans ledit échantillon indique une récupération d'un état septique.

5. Procédé selon la revendication 3, dans lequel un profil d'histones modifié dans l'échantillon mesuré pendant le traitement est indicatif de la réponse du sujet au traitement avec la substance.

6. Utilisation d'un kit comprenant :
a) une ou plusieurs molécules de liaison aux protéines d'histones, choisies dans le groupe constitué de : l'histone H1.4, l'histone H2A, l'histone H2B, l'histone H3, l'histone H4, et des isoformes de celles-ci ; et
(b) une valeur de référence de la quantité des biomarqueurs sélectionnés chez un patient atteint de SRIS pour la comparaison des résultats, pour la prédiction, le pronostic et/ou le diagnostic d'état septique par rapport à SRIS, la molécule de liaison étant de préférence choisie dans le groupe constitué d'anticorps monoclonaux, d'anticorps polyclonaux, d'aptamères, de photo-aptamères, de protéines d'interaction spécifique, et de petites molécules d'interaction spécifique.

7. Procédé selon la revendication 2, comprenant :
(a) l'obtention d'un profil de biomarqueurs candidat d'un échantillon de sang prélevé à partir dudit sujet, dans lequel ledit profil de biomarqueurs candidat est basé sur une ou plusieurs protéines d'histones, choisies dans le groupe constitué de l'histone H1.4, l'histone H2A, l'histone H2B, l'histone H3, l'histone H4, et des isoformes de celles-ci, et
(b) la comparaison dudit profil candidat à un profil de biomarqueur de référence.

8. Procédé selon la revendication 7, dans lequel le profil de biomarqueurs de référence comprend une plage de valeurs normales de biomarqueurs sélectionnés chez des sujets témoins atteints de SRIS, de sorte qu'une augmentation de la quantité des biomarqueurs sélectionnés dans l'échantillon à un taux plus élevé que la plage de valeurs normales de biomarqueurs sélectionnés soit indicatrice d'un état septique, ou dans lequel le profil de biomarqueurs de référence comprend une plage de valeurs de biomarqueurs sélectionnés obtenues à partir de sujets ayant un état septique, de sorte qu'une quantité des biomarqueurs sélectionnés dans ledit échantillon comparable à la plage de valeurs des biomarqueurs sélectionnés chez des sujets atteints d'état septique soit indicatrice d'un état septique.

9. Procédé selon l'une quelconque des revendications précédentes dans lequel l'échantillon est du sérum, ou du plasma.

10. Procédé selon l'une quelconque des revendications précédentes, dans lequel le taux de protéine d'histone ou le profil de biomarqueurs est établi en utilisant une technologie d'immunoessai choisie dans le groupe de technologies ELISA direct, ELISA indirect, ELISA en sandwich, ELISA compétitif, ELISA multiplexe, radioimmunoessai, ou ELISPOT, ou en utilisant des procédés d'analyse par spectrométrie de masse (MS) des protéines présentes dans ledit échantillon, de préférence la surveillance de réactions multiples (Multiple Reaction Monitoring : MRM).
